(19) Europäisches Patentamt
European Patent Office
Office européen des brevets

(11) **EP 1 663 488 B1**

(12) **EUROPÄISCHE PATENTSCHRIFT**

(45) Veröffentlichungstag und Bekanntmachung des Hinweises auf die Patenterteilung:
**06.03.2019 Patentblatt 2019/10**

(21) Anmeldenummer: **04765332.4**

(22) Anmeldetag: **17.09.2004**

(51) Int Cl.:
*B01J 35/02* *(2006.01)*  *B01J 23/887* *(2006.01)*
*C07C 45/35* *(2006.01)*  *B01J 35/10* *(2006.01)*
*B01J 37/08* *(2006.01)*  *B01J 23/888* *(2006.01)*

(86) Internationale Anmeldenummer:
**PCT/EP2004/010436**

(87) Internationale Veröffentlichungsnummer:
**WO 2005/030393 (07.04.2005 Gazette 2005/14)**

(54) **VERFAHREN ZUR HERSTELLUNG VON RINGFÖRMIGEN VOLLKATALYSATOREN**

METHOD FOR THE PRODUCTION OF ANNULAR-SHAPED SUPER CATALYSTS

PROCEDE POUR PRODUIRE DES CATALYSEURS INTEGRAUX ANNULAIRES

(84) Benannte Vertragsstaaten:
**AT BE BG CH CY CZ DE DK EE ES FI FR GB GR HU IE IT LI LU MC NL PL PT RO SE SI SK TR**

(30) Priorität: **22.09.2003 DE 10344149**
**22.09.2003 US 504207 P**

(43) Veröffentlichungstag der Anmeldung:
**07.06.2006 Patentblatt 2006/23**

(73) Patentinhaber: **BASF SE**
**67056 Ludwigshafen am Rhein (DE)**

(72) Erfinder:
• **PETZOLDT, Jochen**
**67273 Weisenheim am Berg (DE)**
• **MÜLLER-ENGEL, Klaus Joachim**
**76297 Stutensee (DE)**

• **UNVERRICHT, Signe**
**68169 Mannheim (DE)**

(74) Vertreter: **BASF IP Association**
**BASF SE**
**G-FLP-C006**
**67056 Ludwigshafen (DE)**

(56) Entgegenhaltungen:
**WO-A-02/062737    DE-A- 3 338 380**
**US-A- 4 366 093    US-A- 5 082 819**

Bemerkungen:
Die Akte enthält technische Angaben, die nach dem Eingang der Anmeldung eingereicht wurden und die nicht in dieser Patentschrift enthalten sind.

EP 1 663 488 B1

**Beschreibung**

Vorliegende Erfindung betrifft ringförmige Vollkatalysatoren mit gekrümmter und/oder nicht gekrümmter Stirnfläche der Ringe, deren Aktivmasse eine Stöchiometrie der allgemeinen Formel I,

$$Mo_{12}Bi_aFe_bX^1_cX^2_dX^3_eX^4_fO_n \qquad (I),$$

mit

X$^1$ =    Nickel und/oder Kobalt,
X$^2$ =    Thallium, ein Alkalimetall und/oder ein Erdalkalimetall,
X$^3$ =    Zink, Arsen, Bor, Antimon, Zinn, Cer, Blei und oder Wolfram,
X$^4$ =    Silicium, Aluminium, Titan und/oder Zirkonium,

a =    0,2 bis 5,
b =    0,01 bis 5,
c =    0 bis 10,
d =    0 bis 2,
e =    0 bis 8,
f =    0 bis 10 und
n =    eine Zahl, die durch die Wertigkeit und Häufigkeit der von Sauerstoff verschiedenen Elemente in I bestimmt wird,

und deren ringförmige Geometrie, ohne Berücksichtigung einer gegebenenfalls bestehenden Krümmung der Stirnfläche, eine Länge L von 2 bis 11 mm, einen Außendurchmesser A von 2 bis 11 mm und eine Wandstärke W von 0,75 mm bis 1,75 mm aufweist,
und die dadurch gekennzeichnet sind, dass die spezifische Oberfläche O 5 bis 20 m$^2$/g und das Porengesamtvolumen V 0,1 bis 1 cm$^3$/g beträgt, wobei die verschiedenen Porendurchmesser wie folgt zu V beitragen:

Poren mit Durchmesser im Bereich < 0,03 $\mu$m: $\leq$ 5 Vol.-%;
Poren mit Durchmesser im Bereich $\geq$ 0,03 bis $\leq$ 0,1 $\mu$m: $\leq$ 25 Vol.-%;
Poren mit Durchmesser im Bereich > 0,1 bis < 1 $\mu$m: $\geq$ 70 Vol.-% und
Poren mit Durchmesser im Bereich $\geq$ 1 bis $\leq$ 10 $\mu$m: $\leq$ 10 Vol.-%,

und die dadurch erhältlich sind, dass man aus Quellen der elementaren Konstituenten der Aktivmasse ein feinteiliges formbares Gemisch erzeugt dessen Körnung zu wenigstens 80 Gew.-% der Gesamtmasse 200 $\mu$m bis 1,5 mm beträgt und aus diesem Gemisch, gegebenenfalls nach Zugabe von Formungs- und/oder Verstärkungshilfsmitteln, ringförmige Vollkatalysatorvorläuferformkörper deren Seitendruckfestigkeit $\geq$12 N und $\leq$ 23 N beträgt formt, deren Stirnflächen gekrümmt und/oder nicht gekrümmt sind, und diese durch thermisches Behandeln bei erhöhten Temperaturen, die die Temperatur 350°C überschreiten und die Temperatur 650°C nicht überschreiten, in die ringförmigen Vollkatalysatoren überführt,
wobei

-    spezifische Oberflächen O nach DIN 66131 durch Gasadsorption (N$_2$) nach Brunauer-Emmet-Teller,
-    Porengesamtvolumina V sowie Durchmesserverteilungen auf diese Porengesamtvolumina mit der Methode der Quecksilberporosimetrie in Anwendung des Gerätes Auto Pore 9220 der Fa. Micromeritics GmbH über eine Bandbreite von 30 Å bis 0,3 mm, und
-    Seitendruckfestigkeiten mit einer Material-Prüfmaschine für quasistatische Beanspruchung des Typs Z 2.5 / TS1S der Firma Zwick GmbH & Co. in D-89079 Ulm mit einer Vorkraft von 0,5 N, einer Vorkraft-Geschwindigkeit von 10 mm/min und einer Prüfgeschwindigkeit von 1,6 mm/min

bestimmt werden.
Außerdem betrifft die vorliegende Erfindung die Verwendung der erfindungsgemäßen ringförmigen Vollkatalysatoren als Katalysatoren mit erhöhter Aktivität und Selektivität für die gasphasenkatalytische Partialoxidation von Propen zu Acrolein sowie von iso-Buten bzw. tert.-Butanol bzw. dessen Methylether zu Methacrolein.
Verfahren zur Herstellung ringförmiger Vollkatalysatoren sind bekannt (vgl. z.B. EP-A 575897, DE-A 3300044, DE-A 19855913, DE-A 10046957, EP-A 1340538, DE-A 19948523, DE-A 4407020 sowie DE-A 10101695). Ebenso ist aus vorgenannten Schriften die Verwendung solcher ringförmigen Vollkatalysatoren als Katalysatoren für die gasphasenkatalytische Partialoxidation von Propen zu Acrolein sowie von iso-Buten bzw. tert.-Butanol bzw. dem Methylether des tert.-Butanols zu Methacrolein bekannt.

Die US-A 5082819 betrifft Mischoxidkatalysatoren für die Oxidation von Propen zu Acrolein, welche zumindest die Elemente Mo, Bi, P, und O sowie Si-haltiges Trägermaterial enthalten und in Form eines Katalysatorkörpers mit bestimmtem Verhältnis zwischen äußerer Oberfläche und Volumen, definierter räumlicher Ausdehnung, Porosität, Dichte, spezifischer Oberfläche, Bruchfestigkeit, Abrieb und Durchströmbarkeit vorliegen.

Hinsichtlich der zur Formung des ringförmigen Vollkatalysatorvorläuferformkörpers anzuwendenden Kräfte schweigen sich die Schriften des Standes der Technik in der Regel aus.

Lediglich die DE-A 10101695 und die DE-A 10121592 lehren diesbezüglich, dass die Kompaktierung (Verdichtung) zu den ringförmigen Vollkatalysatorvorläuferformkörpern so erfolgen solle, dass die Seitendruckfestigkeit der resultierenden ringförmigen Vollkatalysatorvorläuferformkörper 10 N beträgt.

Nachteilig an z.B. der Lehre der DE-A 10101695 ist jedoch, dass die gemäß der Lehre der DE-A 10101695 resultierenden ringförmigen Vollkatalysatoren bei ihrer Verwendung als Katalysatoren für die gasphasenkatalytische Partialoxidation von Propen zu Acrolein bzw. von iso-Buten oder tert.-Butanol (bzw. dessen Methylether) zu Methacrolein sowohl hinsichtlich ihrer Aktivität als auch hinsichtlich der Selektivität der Zielproduktbildung nicht voll zu befriedigen vermögen.

Die Aufgabe der vorliegenden Erfindung bestand daher darin, verbesserte ringförmige Vollkatalysatoren zur Verfügung zu stellen.

Demgemäß wurden wie eingangs beschriebene ringförmige Vollkatalysatoren gefunden, die dadurch gekennzeichnet sind, dass die Formung (Verdichtung) zu ihren ringförmigen Vollkatalysatorvorläuferformkörpern so erfolgt, dass die Seitendruckfestigkeit der resultierenden ringförmigen Vollkatalysatorvorläuferformkörper $\geq$ 12 N und $\leq$ 23 N beträgt.

Vorzugsweise beträgt die Seitendruckfestigkeit der resultierenden ringförmigen Vollkatalysatorvorläuferformkörper $\geq$ 13 N und $\leq$ 22 N bzw. $\geq$ 14 N und $\leq$ 21 N. Ganz besonders bevorzugt beträgt die Seitendruckfestigkeit der resultierenden ringförmigen Vollkatalysatorvorläuferformkörper $\geq$ 15 N und $\leq$ 20 N.

Ferner beträgt die Körnung des zu den ringförmigen Vollkatalysatorvorläuferformkörpern zu formenden feinteiligen formbaren Gemischs erfindungsgemäß vorteilhaft 200 $\mu$m bis 1,5 mm, besonders vorteilhaft 400 $\mu$m bis 1 mm. Es liegen wenigstens 80 Gew.-%, besser wenigstens 90 Gew.-% und besonders vorteilhaft wenigstens 95 oder 98 oder mehr Gew.-% der Gesamtmasse in diesem Körnungsbereich. Als Seitendruckfestigkeit wird dabei in dieser Schrift die Druckfestigkeit bei Stauchung des ringförmigen Vollkatalysatorvorläuferformkörpers senkrecht zur zylindrischen Einhüllenden (d.h., parallel zur Fläche der Ringöffnung) verstanden.

Dabei beziehen sich alle Seitendruckfestigkeiten dieser Schrift auf eine Bestimmung mittels einer Material-Prüfmaschine der Firma Zwick GmbH & Co. (D-89079 Ulm) des Typs Z 2.5 / TS1S. Diese Material-Prüfmaschine ist für quasistatische Beanspruchung mit zügigem, ruhendem, schwellendem oder wechselndem Verlauf konzipiert. Sie ist für Zug-, Druck- und Biegeversuche geeignet. Der installierte Kraftaufnehmer des Typs KAF-TC der Firma A.S.T. (D-01307 Dresden) mit der Herstellnummer 03-2038 wurde dabei entsprechend der DIN EN ISO 7500-1 kalibriert und war für den Messbereich 1-500 N einsetzbar (relative Messunsicherheit: + 0,2 %).

Die Messungen wurden mit folgenden Parametern durchgeführt:

Vorkraft: 0,5 N.
Vorkraft-Geschwindigkeit: 10 mm/min.
Prüfgeschwindigkeit: 1,6 mm/min.

Dabei wurde der obere Stempel zunächst langsam bis kurz vor die Fläche der zylindrischen Einhüllenden des ringförmigen Vollkatalysatorvorläuferformkörpers abgesenkt. Dann wurde der obere Stempel abgestoppt, um anschließend mit der deutlich langsameren Prüfgeschwindigkeit mit minimaler, zu weiterer Absenkung erforderlicher, Vorkraft abgesenkt zu werden.

Die Vorkraft, bei der der Vollkatalysatorvorläuferformkörper Rissbildung zeigt, ist die Seitendruckfestigkeit (SDF).

Erfindungsgemäß besonders vorteilhafte Vollkatalysatorringgeometrien erfüllen zusätzlich die Bedingung L / A = 0,3 bis 0,7. Besonders bevorzugt ist L / A 0,4 bis 0,6.

Weiterhin ist es erfindungsgemäß vorteilhaft, wenn das Verhältnis I / A (wobei I der Innendurchmesser der Vollkatalysatorringgeometrie ist) 0,5 bis 0,8, vorzugsweise 0,6 bis 0,7 beträgt.

Besonders vorteilhaft sind Vollkatalysatorringgeometrien, die gleichzeitig eines der vorteilhaften L / A-Verhältnisse und eines der vorteilten I / A-Verhältnisse aufweisen. Solche möglichen Kombinationen sind z.B. L / A = 0,3 bis 0,7 und I / A = 0,5 bis 0,8 oder 0,6 bis 0,7. Alternativ kann L / A 0,4 bis 0,6 und I / A gleichzeitig 0,5 bis 0,8 oder 0,6 bis 0,7 betragen.

Ferner ist es erfindungsgemäß bevorzugt, wenn L 2 bis 6 mm und besonders bevorzugt, wenn L 2 bis 4 mm beträgt.

Weiterhin ist es vorteilhaft, wenn A 4 bis 8 mm, vorzugsweise 5 bis 7 mm beträgt.

Die Wandstärke der erfindungsgemäß erhältlichen Vollkatalysatorringgeometrien beträgt mit Vorteil 1 bis 1,5 mm.

D.h., erfindungsgemäß günstige Vollkatalysatorringgeometrien sind z.B. solche mit L = 2 bis 6 mm und A = 4 bis 8 mm oder 5 bis 7 mm. Alternativ kann L 2 bis 4 mm und A gleichzeitig 4 bis 8 mm oder 5 bis 7 mm betragen. In allen vorgenannten Fällen kann die Wandstärke W 0,75 bis 1,75 mm oder 1 bis 1,5 mm betragen.

Besonders bevorzugt sind unter den vorgenannten günstigen Vollkatalysatorgeometrien jene, bei denen gleichzeitig die vorstehend genannten L / A sowie I / A Kombinationen erfüllt sind.

Mögliche erfindungsgemäß erhältliche Vollkatalysatorringgeometrien sind somit (A x L x I) 5 mm x 3 mm x 2 mm, oder 5 mm x 3 mm x 3 mm, oder 5,5 mm x 3 mm x 3,5 mm, oder 6 mm x 3 mm x 4 mm, oder 6,5 mm x 3 mm x 4,5 mm, oder 7 mm x 3 mm x 5 mm.

Die Stirnflächen der erfindungsgemäß erhältlichen Ringe können auch entweder beide oder nur eine wie in der EP-A 184790 beschrieben gekrümmt sein und zwar z.B. so, dass der Radius der Krümmung vorzugsweise das 0,4 bis 5-fache des Außendurchmessers A beträgt. Erfindungsgemäß bevorzugt sind beide Stirnflächen ungekrümmt.

Alle diese Vollkatalysatorringgeometrien eignen sich z.B. sowohl für die gasphasenkatalytische Partialoxidation von Propen zu Acrolein als auch für die gasphasenkatalytische Partialoxidation von iso-Buten oder tert.-Butanol oder dem Methylether des tert.-Butanols zu Methacrolein.

Betreffend die Aktivmassen der Stöchiometrie der allgemeinen Formel I betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient a beträgt vorzugsweise 0,4 bis 2. Der stöchiometrische Koeffizient f beträgt vorteilhaft 0,5 oder 1 bis 10. Besonders bevorzugt liegen die vorgenannten stöchiometrischen Koeffizienten gleichzeitig in den genannten Vorzugsbereichen.

Ferner ist $X^1$ vorzugsweise Kobalt, $X^2$ ist vorzugsweise K, Cs und/oder Sr, besonders bevorzugt K, $X^3$ ist bevorzugt Zink und $X^4$ ist bevorzugt Si. Besonders bevorzugt weisen die Variablen $X^1$ bis $X^4$ gleichzeitig die vorgenannten Bedeutungen auf.

Besonders bevorzugt weisen alle stöchiometrischen Koeffizienten a bis f und alle Variablen $X^1$ bis $X^4$ gleichzeitig ihre vorgenannten vorteilhaften Bedeutungen auf.

Als Formungshilfsmittel (Gleitmittel) kommen erfindungsgemäß z.B. Ruß, Stearinsäure, Stärke, Polyacrylsäure, Mineral- oder Pflanzenöl, Wasser, Bortrifluorid oder Graphit in Betracht. Auch Glycerin und Celluloseether können als Gleitmittel eingesetzt werden.

Bezogen auf die zum Vollkatalysatorvorläuferformkörper zu formende Masse werden in der Regel ≤ 5 Gew.-%, meist ≤ 3 Gew.-%, vielfach ≤ 2 Gew.-% an Formungshilfsmittel zugesetzt. Üblicherweise beträgt die vorgenannte Zusatzmenge ≥ 0,5 Gew.-%. Erfindungsgemäß bevorzugtes Gleithilfsmittel ist Graphit.

Im Rahmen der thermischen Behandlung der ringförmigen Vollkatalysatorvorläuferformkörper werden die Formungshilfsmittel meist weitgehend in gasförmige Komponenten zersetzt und/oder verbrannt, so dass der erfindungsgemäß erhältliche ringförmige Vollkatalysator normalerweise teilweise oder vollständig frei von mitverwendeten Formungshilfsmitteln ist. Soweit noch Formungshilfsmittel in den erfindungsgemäß erhältlichen ringförmigen Vollkatalysatoren enthalten ist, verhält es sich bezüglich der durch die Vollkatalysatoren katalysierten Partialoxidationen im Wesentlichen inert.

Letzteres gilt auch für gegebenenfalls vorab der Formgebung zugesetzte feinteilige Verstärkungsmittel wie Mikrofasern aus Glas, Asbest, Siliciumcarbid oder Kaliumtitanat. Die Formung zum ringförmigen Vollkatalysatorvorläuferformkörper kann z.B. mittels einer Tablettierungsmaschine, einer Extrusionsverformungsmaschine oder dergleichen durchgeführt werden.

Die thermische Behandlung des ringförmigen Vollkatalysatorvorläuferformkörpers erfolgt bei Temperaturen, die 350°C überschreiten. Im Rahmen der thermischen Behandlung wird die Temperatur von 650°C jedoch nicht überschritten. Erfindungsgemäß vorteilhaft wird im Rahmen der thermischen Behandlung die Temperatur von 600°C, bevorzugt die Temperatur von 550°C und besonders bevorzugt die Temperatur von 500°C nicht überschritten. Ferner wird beim erfindungsgemäßen Verfahren im Rahmen der thermischen Behandlung des ringförmigen Vollkatalystorvorläuferformkörpers vorzugsweise die Temperatur von 380°C, mit Vorteil die Temperatur von 400°C, mit besonderem Vorteil die Temperatur von 420°C und ganz besonders bevorzugt die Temperatur von 440°C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur von 150 bis 350°C, vorzugsweise 220 bis 280°C, und daran anschließend eine thermische Behandlung bei einer Temperatur von 400 bis 600°C, vorzugsweise 430 bis 550°C durchgeführt werden.

Normalerweise nimmt die thermische Behandlung des ringförmigen Vollkatalysatorvorläuferformkörpers mehrere Stunden (meist mehr als 5 h) in Anspruch. Häufig erstreckt sich die Gesamtdauer der thermischen Behandlung auf mehr als 10 h. Meist werden im Rahmen der thermischen Behandlung des ringförmigen Vollkatalysatorvorläuferformkörpers Behandlungsdauern von 45 h bzw. 25 h nicht überschritten. Oft liegt die Gesamtbehandlungsdauer unterhalb von 20 h. Erfindungsgemäß vorteilhaft werden im Rahmen der erfindungsgemäßen thermischen Behandlung des ringförmigen Vollkatalysatorvorläuferformkörpers 500°C (460°C) nicht überschritten und die Behandlungsdauer im Tempe-

raturfenster von $\geq 400°C$ ($\geq 440°C$) erstreckt sich auf 5 bis 20 h.

Die thermische Behandlung (auch die nachfolgend angesprochene Zersetzungsphase) der ringförmigen Vollkatalysatorvorläuferformkörper kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$ oder Methan, Acrolein, Methacrolein) erfolgen. Selbstredend kann die thermische Behandlung auch unter Vakuum ausgeführt werden.

Prinzipiell kann die thermische Behandlung der ringförmigen Vollkatalysatorvorläuferformkörper in den unterschiedlichsten Ofentypen wie z.B. beheizbare Umluftkammern, Hordenöfen, Drehrohröfen, Bandcalzinierer oder Schachtöfen durchgeführt werden. Erfindungsgemäß bevorzugt erfolgt die thermische Behandlung der ringförmigen Vollkatalysatorvorläuferformkörper in einer Bandcalciniervorrichtung, wie sie die DE-A 10046957 und die WO 02/24620 empfehlen.

Die thermische Behandlung der ringförmigen Vollkatalysatorvorläuferformkörper unterhalb von 350°C verfolgt in der Regel die thermische Zersetzung der in den Vollkatalysatorvorläuferformkörpern enthaltenen Quellen der elementaren Konstituenten des angestrebten ringförmigen Vollkatalysators. Häufig erfolgt beim erfindungsgemäßen Verfahren diese Zersetzungsphase im Rahmen des Aufheizens auf Temperaturen $\geq 350°C$.

Die ringförmigen Vollkatalysatorvorläuferformkörper von erfindungsgemäß erhältlichen ringförmigen Vollkatalysatoren, deren Aktivmasse eine Stöchiometrie der allgemeinen Formel I aufweist, können in erfindungsgemäßer Weise dadurch hergestellt werden, dass man von Quellen der elementaren Konstituenten der Aktivmasse des gewünschten ringförmigen Vollkatalysators ein (möglichst inniges) feinteiliges, der Stöchiometrie der gewünschten Aktivmasse entsprechend zusammengesetztes, formbares Gemisch erzeugt dessen Körnung zu wenigstens 80 Gew.-% der Gesamtmasse 200 $\mu$m bis 1,5 mm beträgt und aus diesem, gegebenenfalls nach Zusatz von Formungs- und/oder Verstärkungshilfsmitteln, einen ringförmigen Vollkatalysatorvorläuferformkörper (mit gekrümmter und/oder nicht gekrümmter Stirnfläche) formt, dessen Seitendruckfestigkeit $\geq 12$ N und $\leq 23$ N beträgt. Die Geometrie des ringförmigen Vollkatalysatorvorläuferformkörpers wird dabei im Wesentlichen derjenigen des gewünschten ringförmigen Vollkatalysators entsprechen.

Als Quellen für die elementaren Konstituenten der gewünschten Aktivmasse kommen solche Verbindungen in Betracht, bei denen es sich bereits um Oxide handelt und/oder um solche Verbindungen, die durch Erhitzen, wenigstens in Abwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu vollständig gasförmig entweichenden Verbindungen zerfallen und/oder zersetzt werden können, können in das feinteilige formbare Gemisch (vorzugsweise ein Trockengemisch) zusätzlich eingearbeitet werden).

Das, vorzugsweise innige, Vermischen der Ausgangsverbindungen (Quellen) zur Herstellung des feinteiligen formbaren Gemischs kann beim Herstellverfahren in trockener oder in nasser Form erfolgen. Erfolgt es in trockener Form, so werden die Ausgangsverbindungen zweckmäßigerweise als feinteilige Pulver eingesetzt. Nach eventuellem Zusatz von Formungs- und/oder Verstärkungshilfsmitteln kann anschließend die Formgebung zum ringförmigen Vollkatalysatorvorläuferformkörper erfolgen.

Erfindungsgemäß bevorzugt erfolgt das innige Vermischen jedoch in nasser Form. Üblicherweise werden dabei die Ausgangsverbindungen in Form einer wässrigen Lösung und/oder Suspension miteinander vermischt. Besonders innige formbare Gemische werden dabei dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene Lösung oder Suspension getrocknet, wobei der Trocknungsprozess vorzugsweise durch Sprühtrocknung mit Austrittstemperaturen von 100 bis 150°C erfolgt.

Das Sprühpulver kann nun gegebenenfalls als solches oder nach Zusatz von Formungs- und/oder Verstärkungshilfsmittel zu den ringförmigen Vollkatalysatorvorläuferformkörpern verdichtet (geformt) werden. Die feinteiligen Verstärkungshilfsmittel können aber auch bereits vorab der Sprühtrocknung (teilweise oder vollständig) zugesetzt werden. Auch kann bei der Trocknung das Lösungs- bzw. Suspendiermittel nur teilweise entfernt werden, falls seine Mitverwendung als Formungshilfsmittel beabsichtigt ist.

Anstatt das Sprühpulver, gegebenenfalls nach Zusatz von Formungs- und/oder Verstärkungshilfsmittel, unmittelbar zu den ringförmigen Vollkatalysatorvorläuferformkörpern (mit gekrümmter und/oder nicht gekrümmter Stirnfläche der Ringe) zu formen, ist es häufig zweckmäßig, zunächst eine Zwischenkompaktierung durchzuführen, um das Pulver zu vergröbern (in der Regel auf eine Körnung von 400 $\mu$m bis 1 mm). Anschließend erfolgt mit dem vergröberten Pulver die eigentliche Ringformung, wobei bei Bedarf zuvor nochmals feinteiliges Gleitmittel zugegeben werden kann.

Als günstiges Gleitmittel für eine solche Zwischenkompaktierung (ebenso wie für die Endformung) hat sich feinteiliges Graphit der Fa. Timcal AG (San Antonio, US) vom Typ TIMREX P44 bzw. Graphitpulver T44 der Fa. Lonza, CH-5643 Sins (Siebanalyse oder Laserbeugung: min. 50 Gew.-% $< 24$ $\mu$m, max. 10 Gew.-% $> 24$ $\mu$m und $\leq 48$ $\mu$m, max. 5 Gew.-% $> 48$ $\mu$m, BET-Oberfläche: 6 bis 13 $m^2$/g) erwiesen. Es fungiert nach der durchgeführten Zwischenkompaktierung gleichzeitig als Gleitmittel bei der eigentlichen Ringformung (und kann bei Bedarf wie beschrieben zuvor zusätzlich ergänzt werden). Es erweist sich als günstig, wenn der Ascherückstand des verwendeten Graphits (Glühen bei 815°C unter Luft) ≤ 0,1 Gew.-% beträgt.

Eine solche Zwischenkompaktierung zum Zweck der Kornvergröberung kann beispielsweise mittels eines Kompaktierers der Fa. Hosokawa Bepex GmbH (D-74211 Leingarten), vom Typ Kompaktor K 200/100 erfolgen. Die Härte des Zwischenkompaktats liegt häufig bereits im Bereich von 10 N. Für die Ringformung zum Vollkatalysatorvorläuferformkörper kommt z.B. ein Kilian Rundläufer (der Fa. Kilian in D-50735 Köln) vom Typ RX 73 oder S 100 in Betracht. Alternativ kann eine Tablettenpresse der Fa. Korsch (D-13509 Berlin) vom Typ PH 800-65 eingesetzt werden.

Alle Angaben in dieser Schrift zu Bestimmungen von spezifischen Oberflächen bzw. von Mikroporenvolumina beziehen sich auf Bestimmungen nach DIN 66131 (Bestimmung der spezifischen Oberfläche von Feststoffen durch Gasadsorption ($N_2$) nach Brunauer-Emmet-Teller (BET)).

Alle Angaben in dieser Schrift zu Bestimmungen von Porengesamtvolumina sowie von Durchmesserverteilungen auf diese Porengesamtvolumina beziehen sich, soweit nichts anderes erwähnt wird, auf Bestimmungen mit der Methode der Quecksilberporosimetrie in Anwendung des Gerätes Auto Pore 9220 der Fa. Micromeritics GmbH, 4040 Neuß, DE (Bandbreite 30 Å bis 0,3 mm).

Erfindungsgemäße ringförmige Vollkatalysatoren sind solche, deren spezifische Oberfläche O 5 bis 20 bzw. 15 m$^2$/g, häufig 5 bis 10 m$^2$/g beträgt. Das Porengesamtvolumen der erfindungsgemäßen ringförmigen Vollkatalysatoren liegt dabei im Bereich von 0,1 bis 1 bzw. 0,8 cm$^3$/g, häufig im Bereich 0,2 bis 0,4 cm$^3$/g.

Im Unterschied zur Lehre der WO 03/039744 sowie zur Lehre der EP-A 279374 tragen die verschiedenen Porendurchmesser bei erfindungsgemäßen ringförmigen Vollkatalysatoren wie folgt zum Porengesamtvolumen bei:

Poren mit Durchmesser im Bereich < 0,03 $\mu$m: ≤ 5 Vol.-%;
Poren mit Durchmesser im Bereich von ≥ 0,03 bis ≤ 0,1 $\mu$m: ≤ 25 Vol.-%;
Poren mit Durchmesser im Bereich von > 0,1 bis < 1 $\mu$m: ≥ 70 Vol.-%; und
Poren mit Durchmesser im Bereich ≥ 1 bis ≤ 10 $\mu$m: ≤ 10 Vol.-%.

D.h., im Unterschied zur Lehre der EP-A 279374, spielt bei erfindungsgemäßen ringförmigen Vollkatalysatoren der Anteil der Poren mit einem Durchmesser ≥ 1 $\mu$m in der Regel nur eine untergeordnete Rolle.

Ferner spielt bei erfindungsgemäßen ringförmigen Vollkatalysatoren der Anteil der Poren mit einem Durchmesser im Bereich von ≥ 0,03 bis ≤ 0,1 $\mu$m in der Regel eine kleinere Rolle.

Besonders vorteilhaft verteilt sich bei erfindungsgemäßen ringförmigen Vollkatalysatoren der Anteil der verschiedenen Porendurchmesser am Porengesamtvolumen wie folgt:

Poren mit Durchmesser im Bereich < 0,03 $\mu$m: ≥ 0 und ≤ 5 Vol.-%, vorzugsweise ≤ 3 Vol.-%;

Poren mit Durchmesser im Bereich von ≥ 0,03 bis ≤ 0,1 $\mu$m: ≥ 3 bzw. ≥ 5 und ≤ 20 bzw. ≤ 15 Vol.-%;

Poren mit Durchmesser im Bereich von > 0,1 bis < 1 $\mu$m: ≥ 75 bzw. ≥ 80 und ≤ 95 bzw. ≤ 90 Vol.-%;

Poren mit Durchmesser im Bereich ≥ 1 $\mu$m bis ≤ 10 $\mu$m: ≥ 0 und ≤ 5 Vol.-%, vorzugsweise ≤ 3 Vol.-%.

D.h., für erfindungsgemäße ringförmige Vollkatalysatoren spielt hinsichtlich deren Performance bei der Verwendung als Katalysatoren zur Partialoxidation von Propen zu Acrolein, bzw. iso-Buten oder tert. Butanol oder Methylether von tert. Butanol zu Methacrolein der Porendurchmesserbereich > 0,1 bis < 1 $\mu$m die entscheidende Rolle.

Im Unterschied dazu fördern Poren im Porendurchmesserbereich von 0,01 bis 0,1 $\mu$m die Partialoxidation von Propen zu Acrylsäure. Dies ist dann vorteilhaft, wenn die Aktivmasse in der ersten Stufe einer zweistufigen Partialoxidation von Propen zu Acrylsäure eingesetzt wird, da in der ersten Stufe gebildete Acrylsäure in der zweiten Stufe weitgehend erhalten bleibt.

Das vorstehende wird auch dadurch zusätzlich bestätigt, dass für erfindungsgemäß besonders vorteilhafte ringförmige Vollkatalysatoren nicht nur die vorstehenden Bedingungen hinsichtlich spezifischer Oberfläche O, Porengesamtvolumen V und Porendurchmesserverteilung erfüllt sind, sondern zusätzlich der prozentual den größten Beitrag zum Porengesamtvolumen V leistende Porendurchmesser d$^{max}$ im Durchmesserbereich 0,3 bis 0,8 $\mu$m, besonders vorteilhaft im Durchmesserbereich 0,4 bis 0,7 $\mu$m und ganz besonders vorteilhaft im Durchmesserbereich 0,5 bis 0,6 $\mu$m liegt.

Erfindungsgemäß überrascht, dass mit zunehmender Seitendruckfestigkeit des ringförmigen Vollkatalysatorvorläuferformkörpers die Porendurchmesser im resultierenden Vollkatalysatorring in der Regel zu größeren Werten verschoben werden.

Dies überrascht insofern, als die Seitendruckfestigkeit des resultierenden ringförmigen Vollkatalysators sich dabei gleichzeitig zu höheren Werten verschiebt. In überraschender Weise ist die Seitendruckfestigkeit des erfindungsgemäß resultierenden ringförmigen Vollkatalysators im Regelfall kleiner als die Seitendruckfestigkeit des zugehörigen ringförmigen Vollkatalysatorvorläuferformkörpers.

In typischer Weise betragen die Seitendruckfestigkeiten von erfindungsgemäß erhältlichen ringförmigen Vollkatalysatoren 5 bis 13 N, häufig 8 bis 11 N. Diese Seitendruckfestigkeiten von erfindungsgemäß erhältlichen ringförmigen Vollkatalysatoren liegen normalerweise auch dann vor, wenn die übrigen als vorteilhaft beschriebenen physikalischen Eigenschaften (z.B. O, V und Porendurchmesserverteilung) von erfindungsgemäß erhältlichen ringförmigen Vollkatalysatoren vorliegen.

Wie bereits erwähnt, eignen sich die erfindungsgemäß erhältlichen ringförmigen Vollkatalysatoren insbesondere als Katalysatoren für die Partialoxidation von Propen zu Acrolein bzw. von iso-Buten und/oder tert. Butanol zu Methacrolein. Die Partialoxidation kann dabei z.B. wie in den Schriften WO 00/53557, WO 00/53558, DE-A 199 10 506, EP-A 1 106 598, WO 01/36364, DE-A 199 27 624, DE-A 199 48 248, DE-A 199 48 523, DE-A 199 48 241, EP-A 700 714, DE-A 10313213, DE-A 10313209, DE-A 10232748, DE-A 10313208, WO 03/039744. EP-A279 374, DE-A 33 38 380, DE-A 33 00 044, EP-A 575 897 sowie DE-A 44 07 020 beschrieben durchgeführt werden, wobei die Katalysatorbeschickung z.B. nur erfindungsgemäß erhältliche ringförmige Vollkatalysatoren oder z.B. mit inerten Formkörpern verdünnte ringförmige Vollkatalysatoren umfassen kann. Im letzteren Fall wird die Katalysatorbeschickung erfindungsgemäß vorteilhaft in der Regel so gestaltet, dass ihre volumenspezifische Aktivität in Strömungsrichtung des Reaktionsgasgemisches kontinuierlich, sprunghaft und/oder stufenförmig zunimmt.

Dabei erweisen sich insbesondere die in dieser Schrift individualisiert hervorgehobenen Ringgeometrien der erfindungsgemäß erhältlichen Vollkatalysatoren dann als vorteilhaft, wenn die Belastung der Katalysatorbeschickung mit im Reaktionsgasausgangsgemisch enthaltenem Propen, iso-Buten und/oder tert. Butanol (bzw. dessen Methylether) ≥ 130 Nl/l Katalysatorbeschickung ·h beträgt (Vor- und/oder Nachschüttungen aus reinem Inertmaterial werden bei Belastungsbetrachtungen nicht als zur Katalysatorbeschickung gehörig betrachtet). Dies insbesondere dann, wenn auch die anderen in dieser Schrift als vorteilhaft beschriebenen physikalischen Eigenschaften von erfindungsgemäß erhältlichen ringförmigen Vollkatalysatoren gegeben sind.

Dieses vorteilhafte Verhalten von erfindungsgemäß erhältlichen ringförmigen Vollkatalysatoren, insbesondere den vorgenannten, liegt aber auch dann vor, wenn die vorgenannte Belastung der Katalysatorbeschickung ≥ 140 Nl/l·h, oder ≥ 150 Nl/l·h, oder ≥ 160 Nl/l·h beträgt. Im Normalfall wird die vorgenannte Belastung der Katalysatorbeschickung ≤ 600 Nl/l·h, häufig ≤ 500 Nl/l·h, vielfach ≤ 400 Nl/l·h oder ≤ 350 Nl/l·h betragen. Belastungen im Bereich von 160 Nl/l·h bis 300 bzw. 250 oder 200 Nl/l·h sind besonders typisch.

Selbstverständlich können die erfindungsgemäß erhältlichen ringförmigen Vollkatalysatoren als Katalysatoren für die Partialoxidation von Propen zu Acrolein bzw. von iso-Buten und/oder tert. Butanol (bzw. dessen Methylether) zu Methacrolein auch bei Belastungen der Katalysatorbeschickung mit der partiell zu oxidierenden Ausgangsverbindung von < 130 Nl/l·h, oder ≤ 120 Nl/l·h, oder ≤ 110 Nl/l·h, betrieben werden. In der Regel wird diese Belastung jedoch bei Werten ≥ 60 Nl/l·h, oder ≥ 70 Nl/l·h, oder ≥ 80 Nl/l·h liegen.

Prinzipiell kann die Belastung der Katalysatorbeschickung mit der partiell zu oxidierenden Ausgangsverbindung (Propen, iso-Buten und/oder tert. Butanol (bzw. dessen Methylether)) über zwei Stellschrauben eingestellt werden:

a) die Belastung der Katalysatorbeschickung mit Reaktionsgasausgangsgemisch; und/oder

b) den Gehalt des Reaktionsgasausgangsgemischs mit der partiell zu oxidierenden Ausgangsverbindung.

Die erfindungsgemäß erhältlichen ringförmigen Vollkatalysatoren eignen sich insbesondere auch dann, wenn bei oberhalb von 130 Nl/l·h liegenden Belastungen der Katalysatorbeschickung mit der partiell zu oxidierenden organischen Verbindung die Belastungseinstellung vor allem über die vorgenannte Stellschraube a) erfolgt.

Der Propenanteil (iso-Butenanteil bzw. tert. Butanolanteil (bzw. dessen Methyletheranteil)) im Reaktionsgasausgangsgemisch wird im Regelfall (d.h. im Wesentlichen unabhängig von der Belastung) 4 bis 20 Vol.-%, häufig 5 bis 15 Vol.-%, oder 5 bis 12 Vol.-%, oder 5 bis 8 Vol.-% betragen (jeweils bezogen auf das Gesamtvolumen).

Häufig wird man das Verfahren der mit den erfindungsgemäß erhältlichen ringförmigen Vollkatalysatoren katalysierten Partialoxidation (im wesentlichen unabhängig von der Belastung) bei einem partiell zu oxidierende (organische) Verbindung (z.B. Propen): Sauerstoff : indifferente Gase (einschließlich Wasserdampf) Volumenverhältnis im Reaktionsgasausgangsgemisch von 1:(1,0 bis 3,0):(5 bis 25), vorzugsweise 1:(1,5 bis 2,3):(10 bis 15) durchführen.

Unter indifferenten Gasen (oder auch Inertgasen) werden dabei solche Gase verstanden, die im Verlauf der Partialoxidation zu wenigstens 95 mol-%, vorzugsweise zu wenigstens 98 mol-% chemisch unverändert erhalten bleiben.

Bei den vorstehend beschriebenen Reaktionsgasausgangsgemischen kann das indifferente Gas zu ≥ 20 Vol.-%, oder zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff bestehen.

Bei Belastungen der Katalysatorbeschickung mit der partiell zu oxidierenden organischen Verbindung von ≥ 250 Nl/l·h ist jedoch die Mitverwendung von inerten Verdünnungsgasen wie Propan, Ethan, Methan, Pentan, Butan,

$CO_2$, CO, Wasserdampf und/oder Edelgasen für das Rektionsgasausgangsgemisch empfehlenswert. Generell können diese inerten Gase und ihre Gemische aber auch bereits bei geringeren erfindungsgemäßen Belastungen der Katalysatorbeschickung mit der partiell zu oxidierenden organischen Verbindung eingesetzt werden. Auch kann Kreisgas als Verdünnungsgas mitverwendet werden. Unter Kreisgas wird das Restgas verstanden, das verbleibt, wenn man aus dem Produktgasgemisch der Partialoxidation die Zielverbindung im Wesentlichen selektiv abtrennt. Dabei ist zu berücksichtigen, dass die Partialoxidationen zu Acrolein oder Methacrolein mit den erfindungsgemäß erhältlichen ringförmigen Vollkatalysatoren nur die erste Stufe einer zweistufigen Partialoxidation zu Acrylsäure oder Methacrylsäure als den eigentlichen Zielverbindungen sein können, so dass die Kreisgasbildung dann meist erst nach der zweiten Stufe erfolgt. Im Rahmen einer solchen zweistufigen Partialoxidation wird in der Regel das Produktgasgemisch der ersten Stufe als solches, gegebenenfalls nach Abkühlung und/oder Sekundärsauerstoffzugabe, der zweiten Partialoxidationsstufe zugeführt.

Bei der Partialoxidation von Propen zu Acrolein, unter Anwendung der erfindungsgemäß erhältlichen ringförmigen Vollkatalysatoren, kann eine typische Zusammensetzung des Reaktionsgasausgangsgemischs (unabhängig von der gewählten Belastung) z.B. die nachfolgenden Komponenten enthalten:

6 bis 6,5 Vol.-% Propen,
3 bis 3,5 Vol.-% $H_2O$,
0,3 bis 0,5 Vol.-% CO,
0,8 bis 1,2 Vol.-% $CO_2$,
0,025 bis 0,04 Vol.-% Acrolein,
10,4 bis 10,7 Vol.-% $O_2$ und
als Restmenge ad 100% molekularer Stickstoff,

oder: 5,4 Vol.-% Propen,

10,5 Vol.-% Sauerstoff,
1,2 Vol.-% $CO_x$,
81,3 Vol.-% $N_2$ und
1,6 Vol.-% $H_2O$.

Das Reaktionsgasausgangsgemisch kann aber auch wie folgt zusammengesetzt sein:

6 bis 15 Vol.-% Propen,
4 bis 30 Vol.-% (häufig 6 bis 15 Vol.-%) Wasser,
$\geq$ 0 bis 10 Vol.-% (vorzugsweise $\geq$ 0 bis 5 Vol.-%) von Propen, Wasser, Sauerstoff und Stickstoff verschiedenen Bestandteilen, soviel molekularem Sauerstoff, dass das molare Verhältnis von enthaltenem molekularem Sauerstoff zu enthaltenem molekularem Propen 1,5 bis 2,5 beträgt und als Restmenge bis zur 100 Vol.-% Gesamtmenge aus molekularem Stickstoff.

Eine andere mögliche Reaktionsgasausgangsgemischzusammensetzung kann enthalten:

6,0 Vol.-% Propen,
60 Vol.-% Luft und
34 Vol.-% $H_2O$.

Alternativ können auch Reaktionsgasausgangsgemische der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 verwendet werden.

Auch eignen sich die erfindungsgemäß erhältlichen ringförmigen Katalysatoren für die Verfahren der DE-A 10246119 bzw. DE-A 10245585.

Weitere erfindungsgemäß geeignete Reaktionsgasausgangsgemische können im nachfolgenden Zusammensetzungsraster liegen:

7 bis 11 Vol.-% Propen,
6 bis 12 Vol.-% Wasser,
$\geq$ 0 bis 5 Vol.-% von Propen, Wasser, Sauerstoff und Stickstoff verschiedenen Bestandteilen,
soviel molekularem Sauerstoff, dass das molare Verhältnis von enthaltenem Sauerstoff zu enthaltenem molekularem Propen 1,6 bis 2,2 beträgt, und

als Restmenge bis zur 100 Vol.-% Gesamtmenge molekularer Stickstoff.

Im Fall von Methacrolein als Zielverbindung kann das Reaktionsgasausgangsgemisch insbesondere auch wie in der DE-A 44 07 020 beschrieben zusammengesetzt sein.

Die Reaktionstemperatur für die Propenpartialoxidation liegt bei Verwendung der erfindungsgemäß erhältlichen ringförmigen Vollkatalysatoren häufig bei 300 bis 380°C. Das gleiche trifft im Fall von Methacrolein als Zielverbindung zu.

Der Reaktionsdruck liegt für die vorgenannten Partialoxidationen in der Regel bei 0,5 bzw. 1,5 bis 3 bzw. 4 bar.

Die Gesamtbelastung der Katalysatorbeschickung mit Reaktionsgasausgangsgemisch beläuft sich bei den vorgenannten Partialoxidationen typisch auf 1000 bis 10000 Nl/l·h, meist auf 1500 bis 5000 Nl/l·h und oft auf 2000 bis 4000 Nl/l·h.

Als im Reaktionsgasausgangsgemisch zu verwendendes Propen kommen vor allem polymer grade Propen und chemical grade Propen in Betracht, wie es z.B. die DE-A 10232748 beschreibt.

Als Sauerstoffquelle wird normalerweise Luft eingesetzt.

Die Partialoxidation in Anwendung der erfindungsgemäß erhältlichen ringförmigen Vollkatalysatoren kann im einfachsten Fall z.B. in einem Einzonen-Vielkontaktrohr-Festbettreaktor durchgeführt werden, wie ihn die DE-A 44 31 957, die EP-A 700 714 und die EP-A 700 893 beschreiben.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Eine typische Kontaktrohrlänge beläuft sich z.B. auf 3,20 m. Anwendungstechnisch zweckmäßig, beläuft sich die im Rohrbündelbehälter untergebrachte Anzahl an Kontaktrohren auf wenigstens 5000, vorzugsweise auf wenigstens 1000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Die Partialoxidation kann aber auch in einem Mehrzonen (z.B. "Zwei-Zonen") - Vielkontaktrohr-Festbettreaktor durchgeführt werden, wie es die DE-A 199 10 506, die DE-A 10313213, die DE-A 10313208 und die EP-A 1 106 598 insbesondere bei erhöhten Belastungen der Katalysatorbeschickung mit der partiell zu oxidierenden organischen Verbindung empfehlen. Eine typische Kontaktrohrlänge im Fall eines Zweizonen-Vielkontaktrohr-Festbettreaktors beträgt 3,50 m. Alles andere gilt im Wesentlichen wie beim Einzonen-Vielkontaktrohr-Festbettreaktor beschrieben. Um die Kontaktrohre, innerhalb derer sich die Katalysatorbeschickung befindet, wird in jeder Temperierzone ein Wärmeaustauschmittel geführt. Als solche eignen sich z.B. Schmelzen von Salzen wie Kaliumnitrat, Kaliumnitrit, Natriumnitrit und/oder Natriumnitrat, oder von niedriger schmelzenden Metallen wie Natrium, Quecksilber sowie Legierungen verschiedener Metalle. Die Fließgeschwindigkeit des Wärmeaustauschmittels innerhalb der jeweiligen Temperierzone wird in der Regel so gewählt, dass die Temperatur des Wärmeaustauschmittels von der Eintrittsstelle in die Temperaturzone bis zur Austrittstelle aus der Temperaturzone um 0 bis 15°C, häufig 1 bis 10°C, oder 2 bis 8°C, oder 3 bis 6°C ansteigt.

Die Eingangstemperatur des Wärmeaustauschmittels, das, über die jeweilige Temperierzone betrachtet, im Gleichstrom oder im Gegenstrom zum Reaktionsgasgemisch geführt werden kann, wird vorzugsweise wie in den Schriften EP-A 1 106 598, DE-A 19948523, DE-A 19948248, DE-A 10313209, EP-A 700 714, DE-A 10313208, DE-A 10313213, WO 00/53557, WO 00/53558, WO 01/36364, WO 00/53557 sowie den anderen in dieser Schrift als Stand der Technik zitierten Schriften empfohlen gewählt. Innerhalb der Temperierzone wird das Wärmeaustauschmittel bevorzugt mäanderförmig geführt. In der Regel verfügt der Vielkontaktrohr-Festbettreaktor zusätzlich über Thermorohre zur Bestimmung der Gastemperatur im Katalysatorbett. In zweckmäßiger Weise wird der Innendurchmesser der Thermorohre und der Durchmesser der innenliegenden Aufnahmehülse für das Thermoelement so gewählt, dass das Verhältnis von Reaktionswärme entwickelndem Volumen zu Wärme abführender Oberfläche bei Thermorohr und Arbeitsrohren gleich ist.

Der Druckverlust sollte bei Arbeitsrohren und Thermorohr, bezogen auf gleiche GHSV, gleich sein. Ein Druckverlustangleich beim Thermorohr kann durch Zusatz von versplittetem Katalysator zu den Katalysatorformkörpern erfolgen. Dieser Ausgleich erfolgt zweckmäßig über die gesamte Thermorohrlänge homogen.

Zur Bereitung der Katalysatorbeschickung in den Kontaktrohren können beim erfindungsgemäßen Verfahren, wie bereits erwähnt, nur erfindungsgemäß erhältliche ringförmige Vollkatalysatoren oder z.B. auch weitgehend homogene Gemische aus erfindungsgemäß erhältlichen ringförmigen Vollkatalysatoren und keine Aktivmasse aufweisenden, sich bezüglich der heterogen katalysierten partiellen Gasphasenoxidation im wesentlichen inert verhaltenden Formkörpern verwendet werden. Als Materialien für solche inerten Formkörper kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder Steatit (z.B. vom Typ C220 der Fa. CeramTec, DE) in Betracht.

Die Geometrie solcher inerter Verdünnungsformkörper kann im Prinzip beliebig sein. D.h., es können beispielsweise Kugeln, Polygone, Vollzylinder oder auch, wie die Katalysatorformkörper, Ringe sein. Häufig wird man als inerte Verdünnungsformkörper solche wählen, deren Geometrie derjenigen der mit ihnen zu verdünnenden Katalysatorformkörper entspricht. Längs der Katalysatorbeschickung kann aber auch die Geometrie des Katalysatorformkörpers gewechselt oder Katalysatorformkörper unterschiedlicher Geometrie in weitgehend homogener Abmischung eingesetzt werden. In einer weniger bevorzugten Vorgehensweise kann auch die Aktivmasse des Katalysatorformkörpers längs der Katalysatorbeschickung verändert werden.

Ganz generell wird, wie bereits erwähnt, die Katalysatorbeschickung mit Vorteil so gestaltet, dass die volumenspezifische (d.h., die auf die Einheit des Volumens normierte) Aktivität in Strömungsrichtung des Reaktionsgasgemisches entweder konstant bleibt oder zunimmt (kontinuierlich, sprunghaft oder stufenförmig).

Eine Verringerung der volumenspezifischen Aktivität kann in einfacher Weise z.B. dadurch erzielt werden, dass man eine Grundmenge von erfindungsgemäß einheitlich hergestellten ringförmigen Vollkatalysatoren mit inerten Verdünnungsformkörpern homogen verdünnt. Je höher der Anteil der Verdünnungsformkörper gewählt wird, desto geringer ist die in einem bestimmten Volumen der Beschickung enthaltene Aktivmasse bzw. Katalysatoraktivität. Eine Verringerung kann aber auch dadurch erzielt werden, dass man die Geometrie der erfindungsgemäß erhältlichen ringförmigen Vollkatalysatoren so verändert, dass die in der Einheit des Ringgesamtvolumens (einschließlich der Ringöffnung) enthaltene Aktivmassenmenge kleiner wird.

Für die heterogen katalysierten Gasphasenpartialoxidationen mit den erfindungsgemäß erhältlichen ringförmigen Vollkatalysatoren wird die Katalysatorbeschickung vorzugsweise entweder auf der gesamten Länge einheitlich mit nur einem Vollkatalysatorring gestaltet oder wie folgt strukturiert. Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Katalysatorbeschickung, ein im Wesentlichen homogenes Gemisch aus erfindungsgemäß erhältlichem ringförmigem Vollkatalysator und inertem Verdünnungsformkörper (wobei beide vorzugsweise im Wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d.h., z.B. auf einer Länge von 2,00 bis 3,00 m, bevorzugt 2,50 bis 3,00 m) entweder eine nur in geringerem Umfang (als im ersten Abschnitt) verdünnte Schüttung des erfindungsgemäß erhältlichen ringförmigen Vollkatalysators, oder, ganz besonders bevorzugt, eine alleinige (unverdünnte) Schüttung desselben ringförmigen Vollkatalysators, der auch im ersten Abschnitt verwendet worden ist. Natürlich kann über die gesamte Beschickung auch eine konstante Verdünnung gewählt werden. Auch kann im ersten Abschnitt nur mit einem erfindungsgemäß erhältlichen ringförmigen Vollkatalysator von geringer, auf seinen Raumbedarf bezogener, Aktivmassendichte und im zweiten Abschnitt mit einem erfindungsgemäß erhältlichen ringförmigen Vollkatalysator mit hoher, auf seinen Raumbedarf bezogener, Aktivmassendichte beschickt werden (z.B. 6,5 mm x 3 mm x 4,5 mm [A x L x l] im ersten Abschnitt, und 5 x 2 x 2 mm im zweiten Abschnitt).

Insgesamt werden bei einer mit den erfindungsgemäß erhältlichen ringförmigen Vollkatalysatoren als Katalysatoren durchgeführten Partialoxidation zur Herstellung von Acrolein oder Methacrolein die Katalysatorbeschickung, das Reaktionsgasausgangsgemisch, die Belastung und die Reaktionstemperatur in der Regel so gewählt, dass beim einmaligen Durchgang des Reaktionsgasgemischs durch die Katalysatorbeschickung ein Umsatz der partiell zu oxidierenden organischen Verbindung (Propen, iso-Butan, tert-Butanol bzw. dessen Methylether) von wenigstens 90 mol-%, oder 92 mol-%, vorzugsweise von wenigstens 95 mol-% resultiert. Die Selektivität der Acrolein- bzw. Methacroleinbildung wird dabei regelmäßig ≥ 94 mol-%, bzw. ≥ 95 mol-%, oder ≥ 96 mol-%, oder ≥ 97 mol-% betragen. In natürlicher Weise werden dabei möglichst geringe Heißpunkttemperaturen angestrebt.

Insgesamt bedingen dabei die erfindungsgemäß erhältlichen ringförmigen Vollkatalysatoren sowohl eine erhöhte Aktivität als auch eine erhöhte Selektivität der Zielproduktbildung.

Abschließend sei festgehalten, dass die erfindungsgemäß erhältlichen ringförmigen Vollkatalysatoren auch ein vorteilhaftes Bruchverhalten bei der Reaktorbefüllung aufweisen. Auch ihr Druckverlustverhalten ist vorteilhaft. Im übrigen eignen sich die erfindungsgemäß erhältlichen ringförmigen Vollkatalysatoren ganz generell als Katalysatoren mit erhöhter Aktivität und Selektivität für gasphasenkatalytische Partialoxidationen organischer Verbindungen wie niederer (z.B. 3 bis 6 (d.h. 3, 4, 5, oder 6) C-Atome enthaltender) Alkane, Alkanole, Alkanale, Alkene und Alkenale zu olefinisch ungesättigten Adehyden und/oder Carbonsäuren, sowie den entsprechenden Nitrilen (Ammoxidation, vor allem von Propen zu Acrylnitril und von 2-Methylpropen bzw. tert.-Butanol (bzw. dessen Methylether) zu Methacrylnitril) sowie für gasphasenkatalytisch oxidative Dehydrierungen organischer Verbindungen (z.B. 3, 4, 5, oder 6 C-Atome enthaltender).

Für das erfindungsgemäße Verfahren besonders vorteilhafte Stöchiometrien sind:

a) $[Bi_2W_2O_9 \times 2WO_3]_{0,5}[Mo_{12}Co_{5,5}Fe_{2,94}Si_{1,59}K_{0,08}O_x]_1$; und
b) $Mo_{12}Co_7Fe_{2,94}Bi_{0,6}Si_{1,59}K_{0,08}O_x$.

Der Wismutgehalt der erfindungsgemäß erhältlichen Aktivmassen kann auch wie in der DE-A 100 63 162 beschrieben eingestellt werden. Dabei wird aus Ausgangsverbindungen der elementaren Konstituenten der angestrebten Aktivmasse eine Lösung oder Suspension erzeugt, die zwar die zur Herstellung der Aktivmasse erforderliche Gesamtmenge der von Bi verschiedenen elementaren Konstituenten, aber nur eine Teilmenge des zur Herstellung der Aktivmasse erforderlichen Bi enthält, die Lösung oder Suspension unter Erhalt einer Trockenmasse getrocknet und die zur Herstellung der Aktivmasse zusätzlich benötigte Restmenge an Bi in Form einer Ausgangsverbindung des Bi wie in der DE-A 100 63 162 beschrieben unter Erhalt eines formbaren Gemischs in diese Trockenmasse eingearbeitet (z.B. wie im Beispiel der DE-A 100 63 162), das formbare Gemisch in erfindungsgemäßer Weise (gegebenenfalls nach Zugabe von Formungs- und/oder Verstärkungshilfsmitteln) zu einem ringförmigen Vollkatalysatorformkörper geformt und dieser durch thermisches Behandeln (z.B. wie im Beispiel der DE-A 100 63 162) in den gewünschten ringförmigen Vollkatalysator überführt. Die Stöchiometrien (insbesondere der Ausführungsbeispiele) und thermischen Behandlungsbedingungen dieser (vorgenannten) Schrift sind erfindungsgemäß gleichfalls besonders geeignet. Dies gilt insbesondere für die Stöchiometrie $Mo_{12}Bi_{1,0}Fe_3Co_7Si_{1,6}K_{0,08}$.

Die Inbetriebnahme einer frischen Katalysatorbeschickung mit erfindungsgemäß erhältlichen ringförmigen Vollkatalysatoren kann wie in der DE-A 10337788 beschrieben erfolgen. In der Regel nehmen Aktivität und Selektivität der Zielproduktbildung anfänglich mit der Betriebsdauer der Katalysatorbeschickung zu. Diese Formierung kann dadurch beschleunigt werden, dass man sie bei im wesentlichen gleichbleibendem Umsatz unter erhöhter Belastung der Katalysatorbeschickung mit Reaktionsgasausgangsgemisch durchführt und nach weitgehend vollzogener Formierung die Belastung auf ihren Zielwert zurücknimmt.

Es überrascht, dass das Verhältnis R von scheinbarer Massendichte zu wahrer Massendichte ρ (so wie es in der EP-A 1340538 definiert ist) bei den erfindungsgemäß erhältlichen ringförmigen Vollkatalysatoren in der Regel > 0,55 beträgt. Meist beträgt $R \leq 0,9$ bzw. $\leq 0,8$ und $\geq 0,6$ bzw. $\geq 0,65$.

$$\text{Dabei ist } R = 1 / (1 + V \cdot \rho).$$

V ist das Porengesamtvolumen.

Beispiele und Vergleichsbeispiele

A) Herstellung von ringförmigen Vollkatalysatoren mit der nachfolgenden Stöchiometrie S1 der Aktivmasse: $Mo_{12}Co_7Fe_{2,94}Bi_{0,6}Si_{1,59}K_{0,08}O_x$.

Bei 60°C wurden 213 kg Ammoniumheptamolybdattetrahydrat (81,5 Gew.-% $MoO_3$) in 600 l Wasser gelöst. In diese Lösung wurden unter Aufrechterhaltung der 60°C 0,97 kg einer 46,8 gew.-%igen wässrigen Kaliumhydroxidlösung von 20°C eingerührt (dabei wurde eine Lösung A erhalten).

Eine zweite Lösung B wurde hergestellt, indem man unter Rühren zu 333,7 kg einer wässrigen Kobalt-(II)-nitratlösung (12,4 Gew.-% Co) bei 30°C 116,25 kg einer 20°C aufweisenden wässrigen Eisen-(III)-nitratlösung (14,2 Gew.-% Fe) gab. Nach beendeter Zugabe wurde noch 30 min. bei 30°C gerührt. Danach wurden bei 60°C 112,3 kg einer 20°C aufweisenden wässrigen Wismutnitratlösung (11,2 Gew.-% Bi) unter Erhalt der Lösung B eingerührt. Innerhalb von 30 min. wurde bei 60°C die Lösung B in die Lösung A eingerührt. 15 min. nach beendetem Einrühren wurden bei 60°C 19,16 kg Kieselsol (der Fa. Dupont, Typ Ludox®, 46,80 Gew.-% $SiO_2$, Dichte: 1,36 bis 1,42 g/cm$^3$, pH = 8,5 bis 9,5, Alkaligehalt max. 0,5 Gew.-%) in die erhaltene Maische gegeben. Unter Aufrechterhaltung der 60°C wurde noch 15 min. nachgerührt. Dann wurde die erhaltene Maische im Gegenstromverfahren sprühgetrocknet (Gaseingangstemperatur: 400 ± 10°C, Gasausgangstemperatur: 140 ± 5°C) wobei ein Sprühpulver erhalten wurde, dessen Glühverlust (3 h bei 600°C unter Luft) 30 % seines Gewichtes betrug. Die Körnung des Sprühpulvers betrug im Wesentlichen einheitlich 30 μm.

In Teilmengen des erhaltenen Sprühpulvers wurden jeweils zusätzlich 1,5 Gew.-% (bezogen auf die Sprühpulvermenge) feinteiliges Graphit (Siebanalyse: min. 50 Gew.-% < 24 μm, max. 10 Gew.-% $\geq$ 24 μm und $\leq$ 48 μm, max. 5 Gew.-% > 48 μm, BET-Oberfläche: 6 bis 13 m$^2$/g) vom Typ TIMREX P44, der Firma Timcal AG (San Antonio, US) eingemischt. Das dabei jeweils resultierende Trockengemisch wurde mittels eines Kompaktors der Fa. Hosokawa Bepex GmbH (D-74211 Leingarten) vom Typ Kompaktor K 200/100 unter den Bedingungen von 2,8 mm Spaltbreite, 1,0 mm Siebweite, 400 μm Siebweite Unterkorn, 60 kN Presssollkraft und 65 bis 70 Upm Schneckendrehzahl durch Vorkompaktieren auf eine im wesentlichen einheitliche Korngröße von 400 μm bis 1 mm vergröbert. Das Kompaktat hatte eine Härte von 10 N.

Das Kompaktat wurde anschließend mit, bezogen auf sein Gewicht, weiteren 2 Gew.-% desselben Graphit vermischt und anschließend in einem Kilian Rundläufer (Tablettiermaschine) vom Typ Rx73, der Fa. Kilian, D-50735 Köln, unter Stickstoffatmosphäre zu ringförmigen Vollkatalysatorvorläuferformkörpern mit nicht gekrümmter Stirnfläche der Geometrie 5 mm x 3 mm x 2 mm (A x L x I) mit unterschiedlicher Seitendruckfestigkeit verdichtet.

Die resultierenden Vollkatalysatorvorläuferformkörper und ihre Seitendruckfestigkeiten waren:

BW 1: 15 N;
BW 2: 20 N;
VW 1: 25 N.

Zur abschließenden thermischen Behandlung wurden jeweils 1900 g der Vollkatalysatorvorläuferformkörper in einer beheizbaren Umluftkammer (0,12 m$^3$ Innenvolumen) aufgeschüttet (2 Nm$^3$ Luft/min.). Anschließend wurde die Temperatur in der Schüttung wie folgt verändert:

- mit 1°C/min. von 25°C auf 160°C erhöht;
- dann 100 min. bei 160°C gehalten;
- danach mit 3°C/min. von 160°C auf 200°C erhöht;
- dann 100 min. bei 200°C gehalten;
- danach mit 2°C/min. von 200°C auf 230°C erhöht;
- dann 100 min. bei 230°C gehalten;
- danach mit 3°C/min. von 230°C auf 270°C erhöht;
- dann 100 min. bei 270°C gehalten;
- danach mit 1°C/min. auf 380°C erhöht;
- dann 4,5 h bei 380°C gehalten;
- danach mit 1°C/min. auf 430°C erhöht;
- dann 4,5 h bei 430°C gehalten;
- danach mit 1°C/min. auf 500°C erhöht;
- dann 9 h bei 500°C gehalten;
- danach innerhalb von 4 h auf 25°C abgekühlt.

Dabei wurden aus den ringförmigen Vollkatalysatorvorläuferformkörpern die nachfolgenden ringförmigen Vollkatalysatoren erhalten (der erste Buchstabe B steht jeweils für Beispiel, der erste Buchstabe V steht für Vergleichsbeispiel):

BVV 1 → BVK 1;
BVV 2 → BVK 2; und
VVV 1 → WK 1.

Die Parameter O, V, der wesentliche, den größten Beitrag zum Porengesamtvolumen leistende, Porendurchmesser d$^{max}$ sowie der prozentuale Anteil derjenigen Porendurchmesser am Porengesamtvolumen, deren Durchmesser > 0,1 und < 1 μm betragen, waren von diesen ringförmigen Vollkatalysatoren wie folgt beschaffen:

BVK 1: O = 6,4 m$^2$/g; V = 0,32 cm$^3$/g; d$^{max}$ = 0,32 μm; V$^{0,1}_1$-% = 91 %.
BVK 2: O = 6,8 m$^2$/g; V = 0,34 cm$^3$/g; d$^{max}$ = 0,36 μm; V$^{0,1}_1$-% = 87 %.

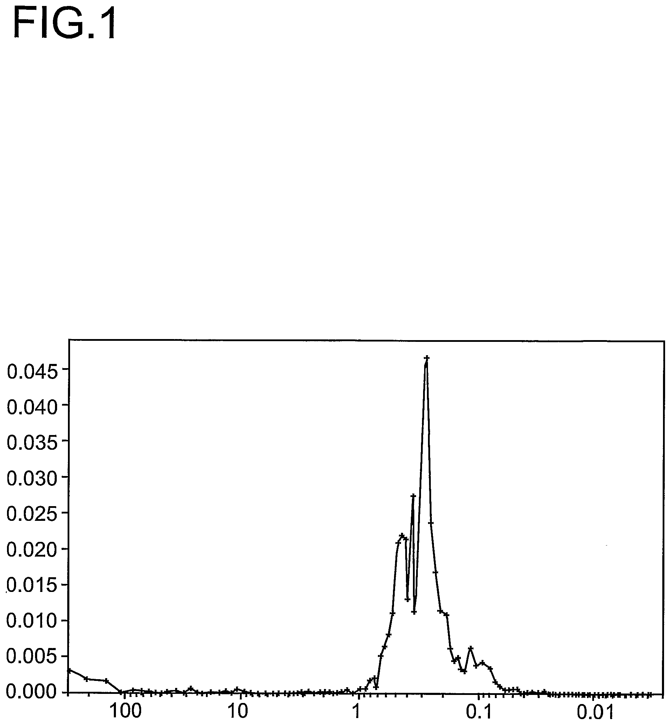

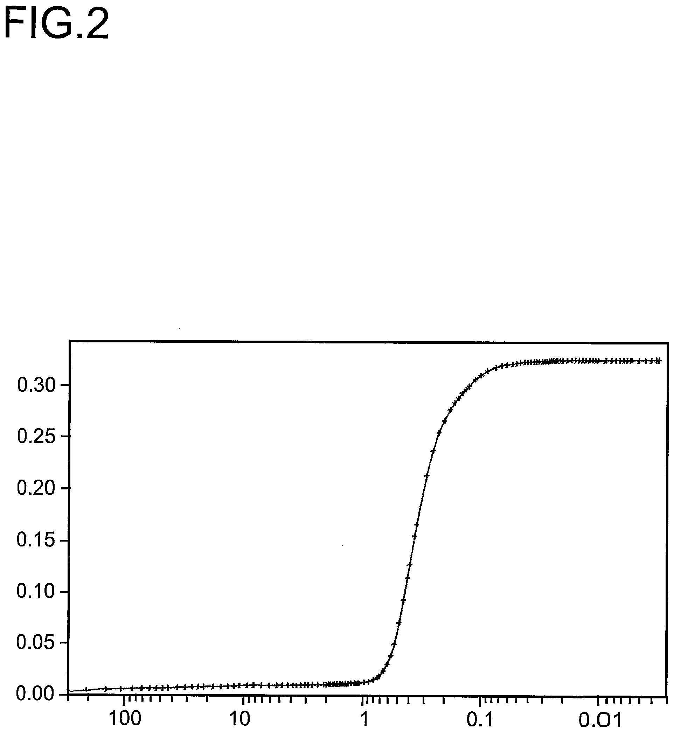

Die Figuren 1 (3) und 2 (4) zeigen außerdem die Porenverteilung des ringförmigen Vollkatalysators BVK1 (BVK2). In Fig. 1(3) zeigt die Abszisse den Porendurchmesser in μm und die Ordinate den differentiellen Beitrag in ml/g des jeweiligen Porendurchmessers zum Porengesamtvolumen. In Fig. 2(4) zeigt die Abszisse ebenfalls den Porendurchmesser in μm und die Ordinate das Integral über die individuellen Beiträge der einzelnen Porendurchmesser zum Porengesamtvolumen in ml/g.

(Anstatt die thermische Behandlung wie beschrieben durchzuführen, kann man sie auch wie in Beispiel 3 der DE-A 10046957 beschrieben mittels einer Bandcalziniervorrichtung durchführen; die Kammern besitzen eine Grundfläche (bei einer einheitlichen Kammerlänge von 1,40 m) von 1,29 m$^2$ (Zersetzung, Kammern 1-4) und 1,40 m$^2$ (Calcination, Kammern 5-8) und werden von unten durch das grobmaschige Band von 75 Nm$^3$/h Zuluft durchströmt, die mittels rotierender Ventilatoren angesaugt werden; innerhalb der Kammern war die zeitliche und örtliche Abweichung der Temperatur vom Sollwert stets ≤ 2°C; durch die Kammern werden die ringförmigen Vollkatalysatorvorläuferformkörper in einer Schichthöhe von 50 mm bis 70 mm geführt; im übrigen wird wie in Beispiel 3 der DE-A 10046957 beschrieben verfahren; die resultierenden ringförmigen Vollkatalysatoren können wie die ringförmigen Vollkatalysatoren BVK1, BVK2 und VVK1 für die nachfolgend unter C) beschriebene gasphasenkatalytische Partialoxidation von Propen zu Acrolein eingesetzt werden).

B) Herstellung von ringförmigen Vollkatalysatoren mit der nachfolgenden Stöchiometrie S2 der Aktivmasse:

$$[Bi_2W_2O_9 \cdot 2WO_3]_{0,5}[Mo_{12}Co_{5,5}Fe_{2,94}Si_{1,59}K_{0,08}O_x]_1.$$

1. Herstellung einer Ausgangsmasse 1

In 775 kg einer wäßrigen salpetersauren Wismutnitratlösung (11,2 Gew.-% Bi; freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wäßrige Gemisch wurde anschließend noch 2 h bei 25°C gerührt und anschließend sprühgetrocknet.

Die Sprühtrocknung erfolgte in einem Drehscheibensprühturm im Gegenstrom bei einer Gaseintrittstemperatur von 300 ± 10°C und einer Gasaustrittstemperatur von 100 ± 10°C. Das erhaltene Sprühpulver (Partikelgröße im Wesentlichen einheitlich 30 $\mu$m), das einen Glühverlust von 12 Gew.-% aufwies (3 h bei 600°C unter Luft glühen), wurde anschließend mit 16,8 Gew.-% (bezogen auf das Pulver) Wasser in einem Kneter angeteigt und mittels eines Extruders (Drehmoment: $\leq$ 50 Nm) zu Strängen des Durchmessers 6 mm extrudiert. Diese wurden in Abschnitte von 6 cm geschnitten, auf einem 3-zonigen Bandtrockner bei einer Verweilzeit von 120 min bei Temperaturen von 90-95°C (Zone 1), 115°C (Zone 2) und 125°C (Zone 3) an Luft getrocknet und dann bei einer Temperatur im Bereich von 780 bis 810°C thermisch behandelt (calciniert; im luftdurchströmten Drehrohrofen (0,3 mbar Unterdruck, 1,54 m$^3$ Innenvolumen, 200 Nm$^3$ Luft/h)). Wesentlich bei der genauen Einstellung der Calcinationstemperatur ist, daß sie an der angestrebten Phasenzusammensetzung des Calcinationsprodukts orientiert zu erfolgen hat. Gewünscht sind die Phasen WO$_3$ (monoklin) und Bi$_2$W$_2$O$_9$, unerwünscht ist das Vorhandensein von $\gamma$-Bi$_2$WO$_6$ (Russellit). Sollte daher nach der Calcination die Verbindung $\gamma$-Bi$_2$WO$_6$ anhand eines Reflexes im Pulverröntgendiffraktogramm bei einem Reflexwinkel von 2$\Theta$ = 28,4° (CuK$\alpha$-Strahlung) noch nachweisbar sein, so ist die Präparation zu wiederholen und die Calcinationstemperatur innerhalb des angegebenen Temperaturbereichs oder die Verweilzeit bei gleichbleibender Calcinationstemperatur zu erhöhen, bis das Verschwinden des Reflexes erreicht wird. Das so erhaltene vorgebildete calcinierte Mischoxid wurde gemahlen, so daß der X$_{50}$-Wert (vgl. Ullmann's Encyclopedia of Industrial Chemistry, 6th Edition (1998) Electronic Release, Kapitel 3.1.4 oder DIN 66141) der resultierenden Körnung 5 mm betrug. Das Mahlgut wurde dann mit 1 Gew.-% (bezogen auf das Mahlgut) feinteiligem SiO$_2$ der Fa. Degussa vom Typ Sipernat® (Rüttelgewicht 150 g/l; X$_{50}$-Wert der SiO$_2$-Partikel betrug 10 $\mu$m, die BET-Oberfläche betrug 100 m$^2$/g) vermischt.

2. Herstellung einer Ausgangsmasse 2

Eine Lösung A wurde hergestellt, indem man bei 60°C unter Rühren in 600 l Wasser 213 kg Ammoniumheptamolybdattetrahydrat (81,5 Gew.-% MoO$_3$) löste und die resultierende Lösung unter Aufrechterhaltung der 60°C und Rühren mit 0,97 kg einer 20°C aufweisenden wässrigen Kaliumhydroxidlösung (46,8 Gew.-% KOH) versetzte.

Eine Lösung B wurde hergestellt indem man bei 60°C in 262,9 kg einer wässrigen Co-(II)-baltnitratlösung (12,4 Gew.-% Co) 116,25 kg einer wäßrigen Eisen-(III)-nitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wäßrigen Gemisch 19,16 kg eines Kieselgels der Fa. Dupont vom Typ Ludox (46,80 Gew.-% SiO$_2$, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gegenstrom sprühgetrocknet (Gaseintrittstemperatur: 400 ± 10°C, Gasaustrittstemperatur: 140 ± 5°C). Das resultierende Sprühpulver wies einen Glühverlust von ca. 30 Gew.-% (3 h bei 600°C unter Luft glühen) und eine im wesentlichen einheitliche Körnung von 30 $\mu$m auf.

3. Herstellung der Multimetalloxidaktivmasse

Die Ausgangsmasse 1 wurde mit der Ausgangsmasse 2 in den für eine Multimetalloxidaktivmasse der Stöchiometrie

$$[Bi_2W_2O_9 \cdot 2WO_3]_{0,5}[Mo_{12}Co_{5,5}Fe_{2,94}Si_{1,59}K_{0,08}O_x]_1$$

erforderlichen Mengen in einem Mischer mit Messerköpfen homogen vermischt. Bezogen auf die vorgenannte Gesamtmasse wurden zusätzlich 1 Gew.-% feinteiliges Graphit der Fa. Timcal AG (San Antonio, US), vom Typ TIMREX P44 (Siebanalyse: min. 50 Gew.-% < 24 $\mu$m, max. 10 Gew.-% $\geq$ 24 $\mu$m und $\leq$ 48 $\mu$m, max. 5 Gew.-% > 48 $\mu$m, BET-

Oberfläche: 6 bis 13 m$^2$/g) homogen eingemischt. Das resultierende Gemisch wurden dann in einem Kompaktor (Fa. Hosokawa Bepex GmbH, D-74211 Leingarten) vom Typ Kompaktor K200/100 mit konkaven, geriffelten Glattwalzen gefahren (Spaltweite: 2,8 mm, Siebweite: 1,0 mm, Siebweite Unterkorn: 400 $\mu$m, Presssollkraft: 60 kN, Schneckendrehzahl: 65 bis 70 Umdrehungen je Minute). Das resultierende Kompaktat wies eine Härte von 10N und eine im wesentlichen einheitliche Körnung von 400 $\mu$m bis 1 mm auf.

Das Kompaktat wurde anschließend mit, bezogen auf sein Gewicht, weiteren 2 Gew.-% desselben Graphit vermischt und anschließend in einem Kilian Rundläufer (Tablettiermaschine) vom Typ R x 73, der Fa. Kilian, D-50735 Köln, unter Stickstoffatmosphäre zu ringförmigen Vollkatalysatorvorläuferformkörpern unterschiedlicher Geometrie (A x L x l) mit unterschiedlicher Seitendruckfestigkeit verdichtet.

Die resultierenden Vollkatalysatorvorläuferformkörper, ihre Geometrien und ihre Seitendruckfestigkeiten waren:

| | | |
|---|---|---|
| BVV3: | 5 mm x 3 mm x 2 mm; | 19 N (Masse: 129 mg). |
| BVV4: | 5 mm x 3 mm x 3 mm; | 16 N. |
| BVV5: | 5 mm x 3 mm x 3 mm; | 17 N. |
| BVV6: | 5,5 mm x 3 mm x 3,5 mm; | 14 N. |
| BVV7: | 5,5 mm x 3 mm x 3,5 mm; | 15,5 N. |
| BVV8: | 6 mm x 3 mm x 4 mm; | 13 N. |
| BVV9: | 6 mm x 3 mm x 4 mm; | 16,3 N. |
| VVV2: | 6,5 mm x 3 mm x 4,5 mm; | 11 N. |
| BVV10: | 6,5 mm x 3 mm x 4,5 mm; | 15,6 N. |
| VVV3: | 7 mm x 3 mm x 5 mm; | 11,7 N. |
| BW11: | 7 mm x 3 mm x 5 mm; | 16,3 N. |
| VVV4: | 5 mm x 3 mm x 2 mm; | 10,5 N |

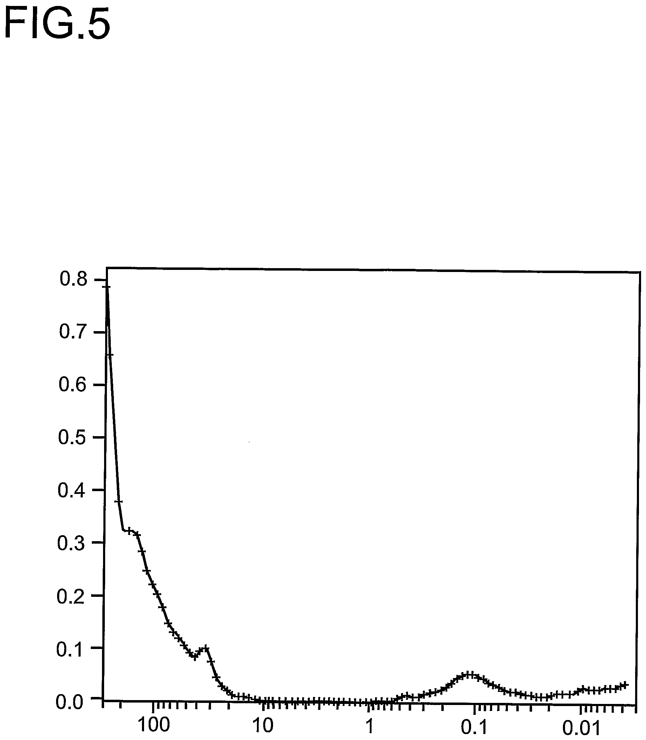

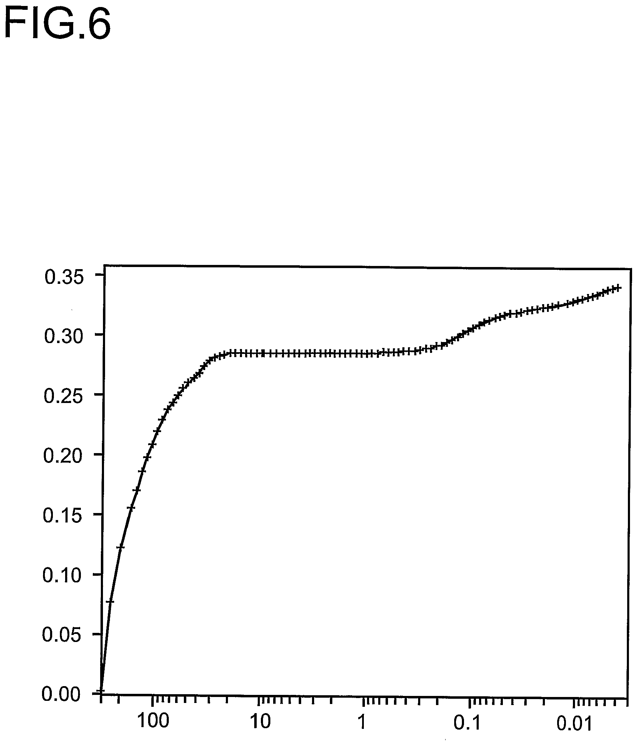

Fig. 5(6) zeigt die Porenverteilung im ringförmigen Vollkatalysatorvorläuferformkörper BW3. Die Achsenbeschriftung von Fig. 5 entspricht derjenigen von Fig. 1 und die Achsenbeschriftung von Fig. 6 entspricht derjenigen von Fig. 2.

Zur abschließenden thermischen Behandlung wurden jeweils 1000 g der Vollkatalysatorvorläuferformkörper in einem Luft durchströmten Muffelofen (60 l Innenvolumen, 1 l/h Luft pro Gramm Vollkatalysatorvorläuferformkörper) zunächst mit einer Aufheizrate von 180°C/h von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h, auf 230°C erhöht wurden. Diese Temperatur wurde ebenfalls 1 h aufrechterhalten, bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im Wesentlichen abgeschlossen. Dann wurde mit einer Aufheizrate von 180°C/h auf 465°C erhitzt und diese Calcinationstemperatur während 4 h aufrechterhalten.

Dabei wurden aus den ringförmigen Vollkatalysatorvorläuferformkörpern die nachfolgenden ringförmigen Vollkatalysatoren erhalten (der erste Buchstabe B steht jeweils für Beispiel, der erste Buchstabe V steht jeweils für Vergleichsbeispiel):

| | O [m$^2$/g] | V [cm$^3$/g] | d$^{max}$ [$\mu$m] | V$^{0,1}_1$-% | R |
|---|---|---|---|---|---|
| BVV3 → BVK3 | 7,6 | 0,27 | 0,6 | 79 | 0,66 |
| BVV4 → BVK4 | 6,9 | 0,23 | 0,45 | 70 | - |
| BVV5 → BVK5 | - | - | - | - | - |
| BVV6 → BVK6 | 7,45 | 0,21 | 0,40 | 74 | - |
| BVV7 → BVK7 | 7,95 | 0,205 | 0,39 | 73 | 0,68 |
| BVV8 → BVK8 | 7,6 | 0,22 | 0,45 | 74 | - |
| BVV9 → BVK9 | 9,61 | 0,22 | 0,30 | 70 | 0,68 |
| VVV2 → VVK2 | - | - | - | - | - |
| BVV10 → BVK10 | - | - | - | - | - |
| VVV3 → VVK3 | - | - | - | - | - |
| BVV11 → BVK11 | - | - | - | - | - |

(fortgesetzt)

| | O [m$^2$/g] | V [cm$^3$/g] | d$^{max}$ [$\mu$m] | V$^{0,1}_1$-% | R |
|---|---|---|---|---|---|
| VVV4 → VVK4 | 10,2 | 0,19 | 0,28 | 64 | - |

Zusätzlich enthält die vorstehende Tabelle Werte für die spezifische Oberfläche O, das Porengesamtvolumen V, den Porendurchmesser d$^{max}$ der den größten Beitrag zum Porengesamtvolumen leistet, sowie die prozentualen Anteile derjenigen Porendurchmesser am Porengesamtvolumen, deren Durchmesser > 0,1 und < 1 $\mu$m betragen und R-Werte.

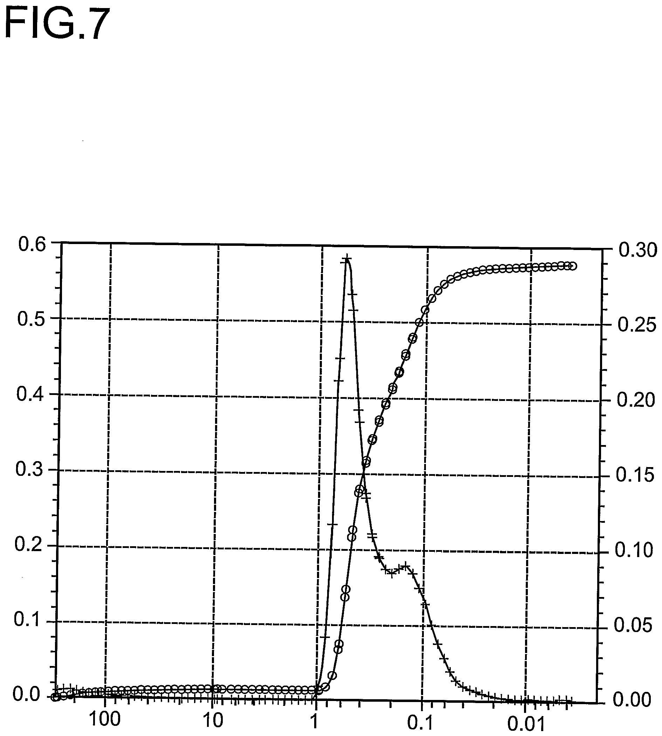

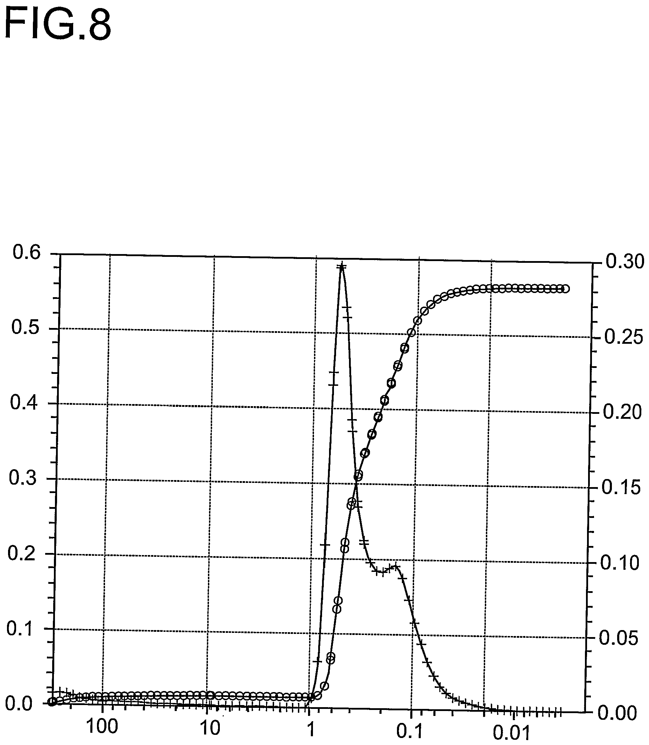

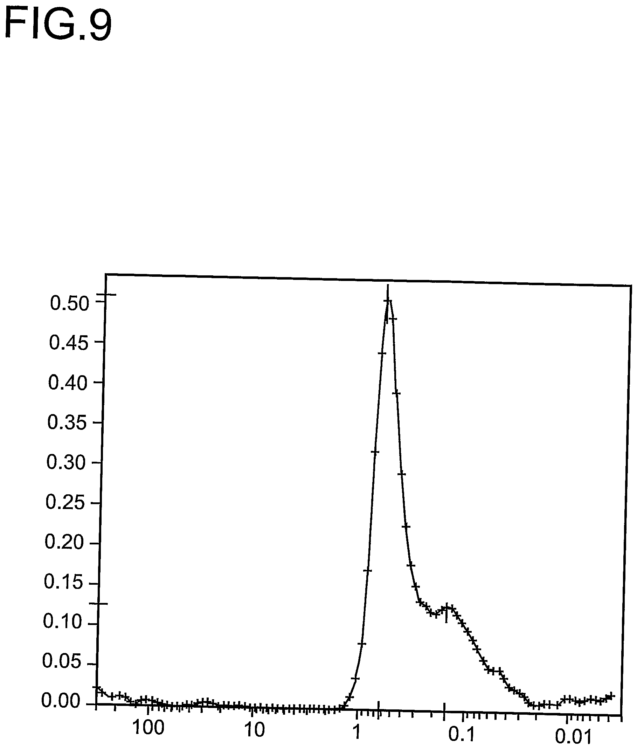

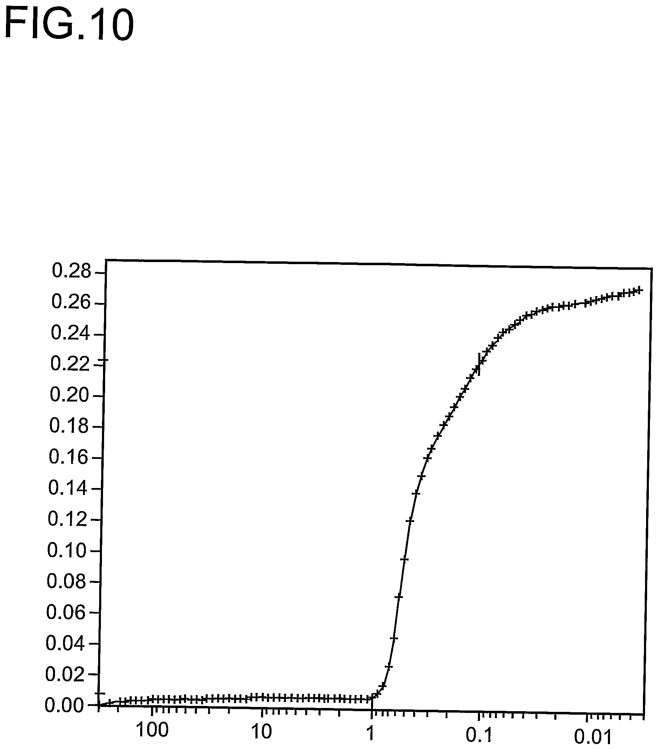

Die Figuren 7 und 8 zeigen außerdem die Porenverteilung des ringförmigen Vollkatalysators BVK3 für zwei voneinander unabhängige Reproduktionen. Auf der Abszisse ist der Porendurchmesser in $\mu$m aufgetragen. Auf der linken Ordinate ist der Logarithmus des differentiellen Beitrags in ml/g des jeweiligen Porendurchmessers zum Porengesamtvolumen aufgetragen (Kurve +). Das Maximum weist den Porendurchmesser mit dem größten Beitrag zum Porengesamtvolumen aus. Auf der rechten Ordinate ist in ml/g das Intergal über die individuellen Beiträge der einzelnen Porendurchmesser zum Porengesamtvolumen aufgetragen (Kurve O). Der Endpunkt ist das Porengesamtvolumen. Die Figuren 9 und 10 zeigen die Porenverteilung einer weiteren Reproduktion des BVK3 bei gleicher Achsenbeschriftung wie in Fig. 7, 8.

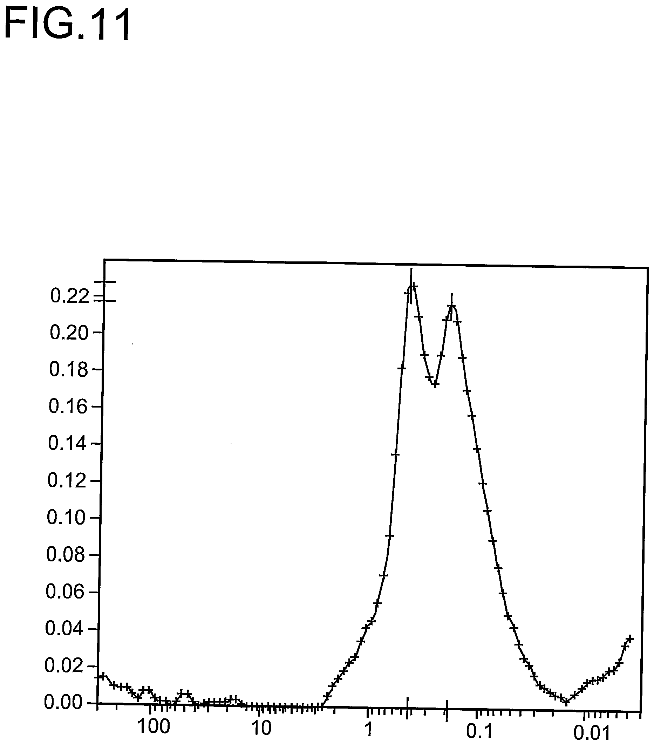

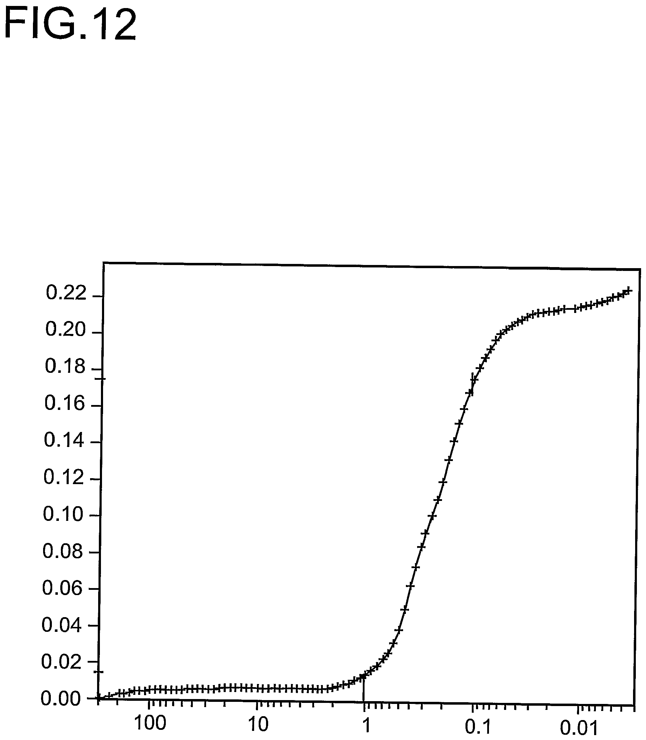

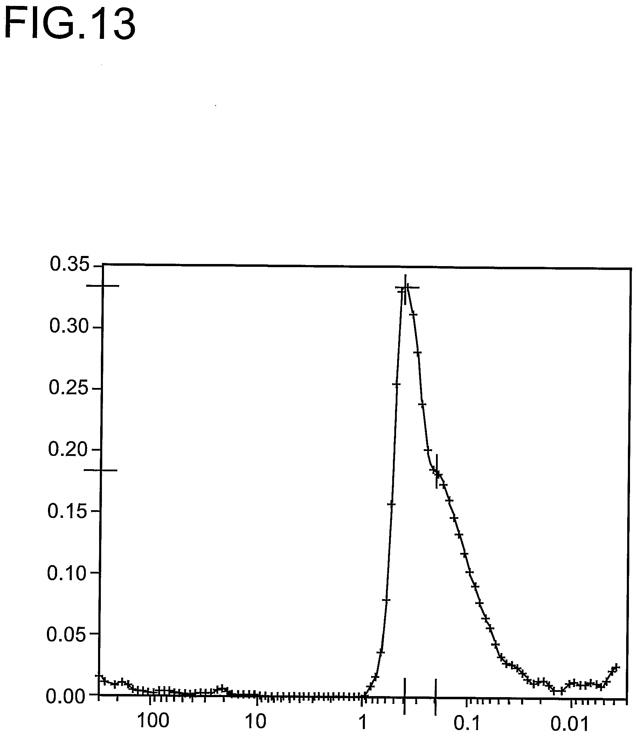

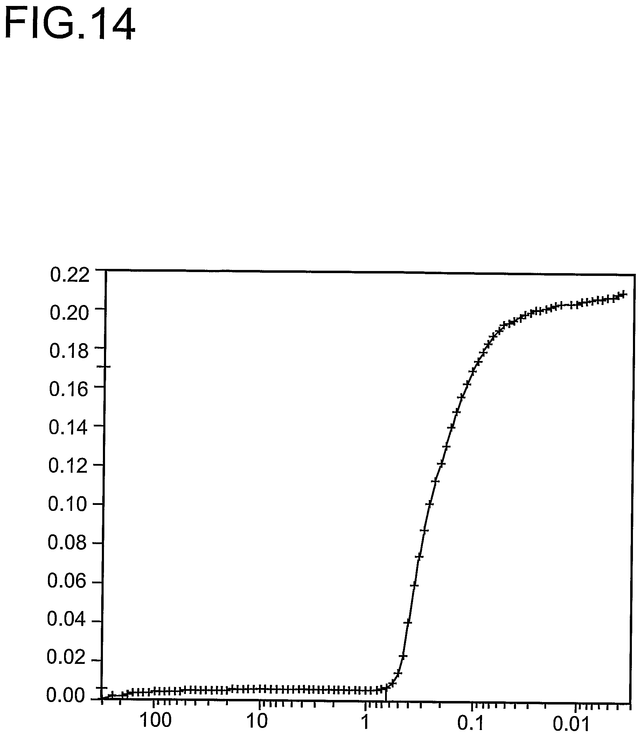

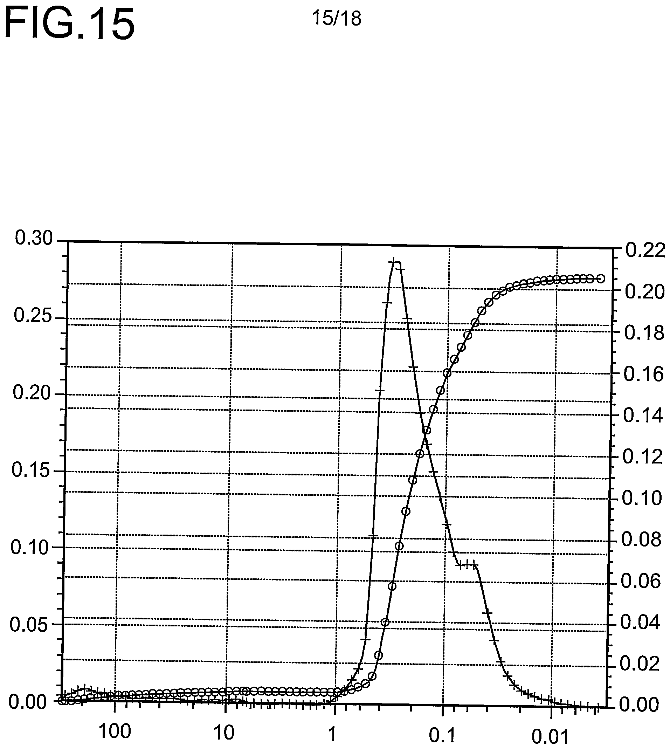

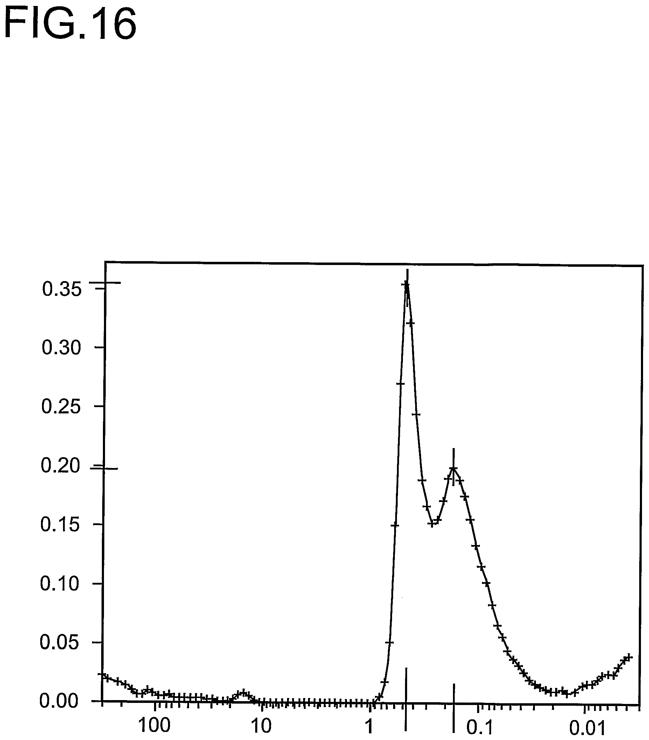

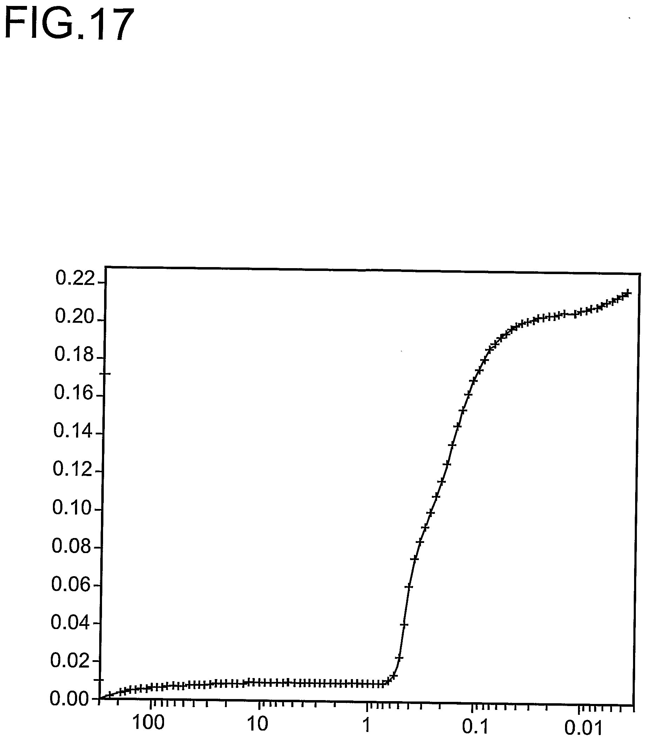

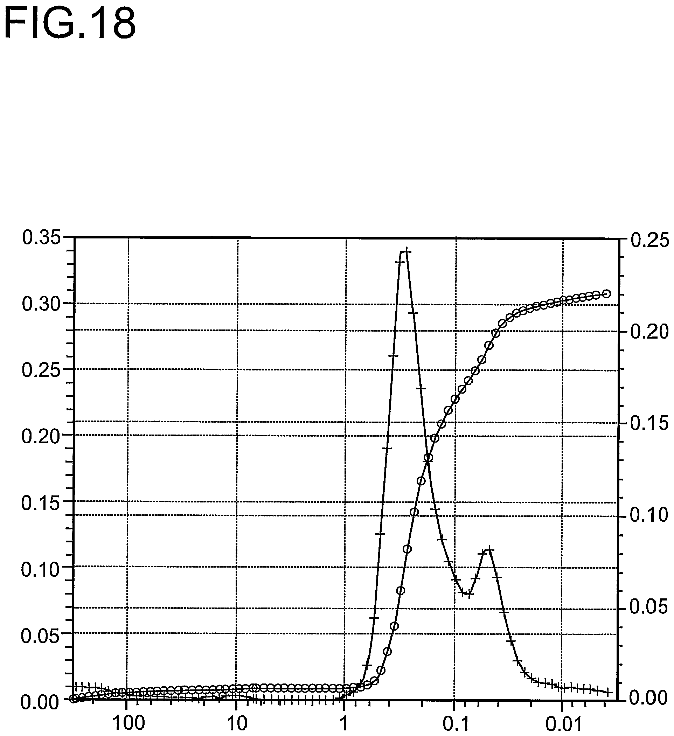

Entsprechende Figuren bilden die Figuren 11, 12 (BVK4), die Figuren 13, 14 (BVK6), die Figur 15 (BVK7), die Figuren 16, 17 (BVK8) und die Figur 18 (BVK9).

Anstatt die thermische Behandlung wie beschrieben durchzuführen, kann man sie auch wie in Beispiel 1 der DE-A 10046957 (die Schütthöhe in der Zersetzung (Kammern 1 bis 4) beläuft sich dabei jedoch vorteilhaft auf 44 mm bei einer Verweilzeit pro Kammer von 1,46 h und in der Calcination (Kammern 5 bis 8) beläuft sie sich vorteilhaft auf 130 mm bei einer Verweilzeit von 4,67 h) beschrieben mittels einer Bandcalziniervorrichtung durchführen; die Kammern besitzen eine Grundfläche (bei einer einheitlichen Kammerlänge von 1,40 m) von 1,29 m$^2$ (Zersetzung) und 1,40 m$^2$ (Calcination) und werden von unten durch das grobmaschige Band von 75 Nm$^3$/h Zuluft durchströmt, die mittels rotierender Ventilatoren angesaugt werden. Innerhalb der Kammern ist die zeitliche und örtliche Abweichung der Temperatur vom Sollwert stets $\leq$ 2°C. Im übrigen wird wie in Beispiel 1 der DE-A 10046957 beschrieben verfahren. Die resultierenden ringförmigen Vollkatalysatoren können wie die ringförmigen Vollkatalysatoren BVK3 bis BVK4 für die nachfolgend beschriebene gasphasenkatalytische Partialoxidation von Propen zu Acrolein eingesetzt werden.

Als weitere Alternative kann die thermische Behandlung in einem Umluftofen (z.B. in einem solchen der Fa. Elino vom Typ Laborkammerofen KA-040/006-08 EW.OH bzw. einem solchen der Fa. Heraeus vom Typ K 750) so durchgeführt werden, dass man innerhalb von 6 h auf 270°C erwärmt und anschließend so lange die 270°C aufrechterhält, bis die Umluft frei ist von nitrosen Gasen. Nachfolgend wird innerhalb von 1,5 h auf eine Temperatur von 430°C bis 460°C (vorzugsweise auf 438°C) erwärmt und diese Temperatur 10 h aufrechterhalten. Die Luftspülung beträgt 800 Nl/h. 1000 g ringförmige Vollkatalysatorvorläuferformkörper sind in einen quaderförmigen Drahtkorb (10 cm hoch, 14 cm x 14 cm Grundfläche) in einer Schütthöhe von ca. 4 cm eingefüllt. Die Restgrundfläche des Tragekorbes wird in entsprechender Schütthöhe mit Steatitringen (wie immer in den Beispielen und Vergleichsbeispielen vom Typ C220 der Fa. Ceram Tec, DE) gleicher Geometrie belegt.

Diese thermischen Behandlungsbedingungen können auch auf die ringförmigen Vollkatalysatorvorläuferformkörper BVV1, BVV2 und VVV1 angewendet werden. Alle resultierenden ringförmigen Vollkatalysatoren können in der unter C) beispielhaft beschriebenen gasphasenkatalytischen Partialoxidation eingesetzt werden.

C) Testung der in A) und B) hergestellten ringförmigen Vollkatalysatoren für eine heterogen katalysierte Partialoxidation von Propen zu Acrolein

1. Versuchsanordnung

Ein Reaktionsrohr (V2A Stahl; 21 mm Außendurchmesser, 3 mm Wandstärke, 15 mm Innendurchmesser, Länge 100 cm) wurde von oben nach unten in Strömungsrichtung wie folgt beschickt:

Abschnitt 1: 30 cm Länge
Steatitringe der Geometrie 5 mm x 3 mm x 2 mm (Außendurchmesser x Länge x Innendurchmesser) als Vorschüttung.

Abschnitt 2: 70 cm Länge
Katalysatorbeschickung mit den in A) und B) hergestellten ringförmigen Vollkatalysatoren.

Die Temperierung des Reaktionsrohres erfolgte mittels eines mit Stickstoff geperlten Salzbades.

2. Versuchsdurchführung

Die beschriebene, jeweils frisch hergerichtete, Versuchsanordnung wurde jeweils kontinuierlich mit einem Beschickungsgasgemisch (Gemisch aus Luft, polymer grade Propylen und Stickstoff) der Zusammensetzung:

5 Vol.-% Propen,
10 Vol.-% Sauerstoff und
als Restmenge bis 100 Vol.-% $N_2$

beschickt, wobei die Belastung und die Thermostatisierung des Reaktionsrohres so erfolgte, dass der Propenumsatz U (mol-%) bei einmaligem Durchgang des Beschickungsgasgemisches durch das Reaktionsrohr kontinuierlich etwa 95 mol-% betrug.

Die nachfolgende Tabelle zeigt die in Abhängigkeit von der gewählten Katalysatorbeschickung und Propenbelastung (PL in Nl/l • h) derselben zur Umsatzerlangung erforderlichen Salzbadtemperaturen $T_S$ (°C) sowie die dabei erzielten Acroleinselektivitäten $S^A$ (mol-%). Die angegebenen Ergebnisse beziehen sich stets auf das Ende einer Betriebsdauer von 120 h. Die Selektivität $S^{AA}$ der Acrylsäurenebenproduktbildung lag im Bereich von 4 bis 17 mol-%.

| ringförmiger Vollkatalysator | PL | $T_S$ | $S^A$ | $S^{AA}$ |
|---|---|---|---|---|
| BVK1 | 50 | 306 | 89,5 | 4,7 |
| BVK2 | 50 | 306 | 89,5 | 4,6 |
| VVK1 | 50 | 309 | 89,0 | 4,0 |
| BVK1 | 75 | 310 | 90,5 | 4,9 |
| BVK2 | 75 | 311 | 90,5 | 4,9 |
| VVK1 | 75 | 313 | 89,8 | 4,5 |
| BVK1 | 100 | 315 | 90,8 | 5,2 |
| BVK2 | 100 | 318 | 91,1 | 5,1 |
| VVK1 | 100 | 321 | 90,5 | 4,8 |
| BVK3 | 50 | 320 | 88,6 | 7,1 |
| BVK4 | 50 | 325 | 86,1 | 8,8 |
| BVK5 | 50 | 322 | 86,6 | 8,9 |
| BVK6 | 50 | 338 | 84,9 | 10,2 |
| BVK7 | 50 | 320 | 90,2 | 5,1 |
| BVK8 | 50 | 343 | 85,0 | 10,3 |
| BVK9 | 50 | 322 | 90,0 | 5,4 |
| VVK2 | 50 | 349 | 83,4 | 12,3 |
| BVK10 | 50 | 333 | 93,1 | 5,4 |
| VVK3 | 50 | 345 | 75,3 | 16,2 |
| BVK11 | 50 | 333 | 87,9 | 7,6 |
| VVK4 | 50 | 337 | 84,3 | 8,6 |

Die vorstehenden Versuche können aber auch in entsprechender Weise (gleicher Zielumsatz) in einem Reaktionsrohr der nachfolgenden Art durchgeführt werden: V2A Stahl; 30 mm Außendurchmesser, 2 mm Wandstärke, 26 mm Innendurchmesser, 350 cm Länge, ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann.

In Strömungsrichtung wird dabei wie folgt beschickt:

Abschnitt 1:   80 cm Länge

Steatitringe der Geometrie 7 mm x 7 mm x 4 mm (Außendurchmesser x Länge x Innendurchmesser) als Vorschüttung.

Abschnitt 2:   270 cm Länge

Katalysatorbeschickung mit den in A) und B) hergestellten ringförmigen Vollkatalysatoren.

Die Temperierung des Reaktionsrohres erfolgt mittels eines im Gegenstrom gepumpten Salzbades.

PL wird durchgehend zu 100 gewählt. Die Zusammensetzung des Reaktionsgasausgangsgemisches ist 5,4 Vol.-% Propen, 10,5 Vol.-% Sauerstoff, 1,2 Vol.-% $CO_x$, 81,3 Vol.-% $N_2$ und 1,6 Vol.-% $H_2O$.

Diese Versuchsdurchführung kann in entsprechender Weise auch mit einer Katalysatorbeschickung durchgeführt werden, deren Abschnitt 2 wie folgt gestaltet ist (jeweils in Strömungsrichtung):

I. Zunächst auf 100 cm Länge ein homogenes Gemisch aus 65 Gew.-% BVK3 und 35 Gew.-% Steatitringen (5 mm x 3 mm x 2 mm);
anschließend auf 170 cm Länge ein homogenes Gemisch aus 90 Gew.-% BVK3 und 10 Gew.-% Steatitringen (5 mm x 3 mm x 2 mm);
oder

II. Zunächst auf 100 cm Länge BVK10;
anschließend auf 170 cm Länge BVK3;
oder

III. Zunächst auf 100 cm Länge ein homogenes Gemisch aus 70 Gew.-% BVK3 und 30 Gew.-% Steatitringen (5 mm x 3 mm x 2 mm); anschließend auf 170 cm Länge BVK3.

$T_s$ wird in allen Fällen so gewählt, dass U-Propen = 95 mol-%.

**Patentansprüche**

**1.** Ringförmiger Vollkatalysator mit gekrümmter und/oder nicht gekrümmter Stirnfläche des Rings, dessen Aktivmasse eine Stöchiometrie der allgemeinen Formel I,

$$Mo_{12}Bi_aFe_bX^1_cX^2_dX^3_eX^4_fO_n \qquad (I),$$

mit

$X^1$ = Nickel und/oder Kobalt,
$X^2$ = Thallium, ein Alkalimetall und/oder ein Erdalkalimetall,
$X^3$ = Zink, Arsen, Bor, Antimon, Zinn, Cer, Blei und/oder Wolfram,
$X^4$ = Silicium, Aluminium, Titan und/oder Zirkonium,
a = 0,2 bis 5,
b = 0,01 bis 5,
c = 0 bis 10,
d = 0 bis 2,
e = 0 bis 8,
f = 0 bis 10 und
n = eine Zahl, die durch die Wertigkeit und Häufigkeit der von Sauerstoff verschiedenen Elemente in I bestimmt wird,

und dessen ringförmige Geometrie, ohne Berücksichtigung einer gegebenenfalls bestehenden Krümmung der Stirnfläche, eine Länge L von 2 bis 11 mm, einen Außendurchmesser A von 2 bis 11 mm und eine Wandstärke W von 0,75 mm bis 1,75 mm aufweist,
und der **dadurch gekennzeichnet ist, dass** die spezifische Oberfläche O 5 bis 20 $m^2/g$ und das Porengesamtvolumen V 0,1 bis 1 $cm^3/g$ beträgt, wobei die verschiedenen Porendurchmesser wie folgt zu V beitragen:

Poren mit Durchmesser im Bereich < 0,03 $\mu$m: $\leq$ 5 Vol.-%;

Poren mit Durchmesser im Bereich $\geq 0,03$ bis $\leq 0,1$ $\mu$m: $\leq 25$ Vol.-%;

Poren mit Durchmesser im Bereich $> 0,1$ bis $< 1$ $\mu$m: $\geq 70$ Vol.-% und

Poren mit Durchmesser im Bereich $\geq 1$ bis $\leq 10$ $\mu$m: $\leq 10$ Vol.-%,

und der dadurch erhältlich ist, dass man aus Quellen der elementaren Konstituenten der Aktivmasse ein feinteiliges formbares Gemisch erzeugt dessen Körnung zu wenigstens 80 Gew.-% der Gesamtmasse 200 $\mu$m bis 1,5 mm beträgt und aus diesem Gemisch, gegebenenfalls nach Zugabe von Formungs- und/oder Verstärkungshilfsmitteln, ringförmige Vollkatalysatorvorläuferformkörper deren Seitendruckfestigkeit $\geq 12$ N und $\leq 23$ N beträgt formt, deren Stirnflächen gekrümmt und/oder nicht gekrümmt sind, und diese durch thermisches Behandeln bei erhöhten Temperaturen, die die Temperatur 350°C überschreiten und die Temperatur 650°C nicht überschreiten, in die ringförmigen Vollkatalysatoren überführt,

wobei

- spezifische Oberflächen O nach DIN 66131 durch Gasadsorption ($N_2$) nach Brunauer-Emmet-Teller,
- Porengesamtvolumina V sowie Durchmesserverteilungen auf diese Porengesamtvolumina mit der Methode der Quecksilberporosimetrie in Anwendung des Gerätes Auto Pore 9220 der Fa. Micromeritics GmbH über eine Bandbreite von 30 Å bis 0,3 mm, und
- Seitendruckfestigkeiten mit einer Material-Prüfmaschine für quasistatische Beanspruchung des Typs Z 2.5 / TS1S der Firma Zwick GmbH & Co. in D-89079 Ulm mit einer Vorkraft von 0,5 N, einer Vorkraft-Geschwindigkeit von 10 mm/min und einer Prüfgeschwindigkeit von 1,6 mm/min

bestimmt werden.

2. Ringförmiger Vollkatalysator nach Anspruch 1, mit O = 5 bis 10 $m^2$/g.

3. Ringförmiger Vollkatalysator nach Anspruch 1 oder 2, mit V = 0,2 bis 0,4 $cm^3$/g.

4. Ringförmiger Vollkatalysator nach einem der Ansprüche 1 bis 3, wobei die verschiedenen Porendurchmesser wie folgt zu V beitragen:

Poren mit Durchmesser im Bereich $< 0,03$ $\mu$m: $\geq 0$ Vol.-% und $\leq 5$ Vol.-%;

Poren mit Durchmesser im Bereich $\geq 0,03$ bis $\leq 0,1$ $\mu$m: $\geq 3$ und $\leq 20$ Vol.-%;

Poren mit Durchmesser im Bereich $> 0,1$ bis $< 1$ $\mu$m: $\geq 75$ und $\leq 95$ Vol.-% und

Poren mit Durchmesser im Bereich $\geq 1$ bis $\leq 10$ $\mu$m: $\geq 0$ und $\leq 5$ Vol.-%.

5. Ringförmiger Vollkatalysator nach einem der Ansprüche 1 bis 4, wobei der den größten Beitrag zum Porengesamtvolumen V leistende Porendurchmesser $d^{max}$ 0,3 bis 0,8 $\mu$m beträgt.

6. Ringförmiger Vollkatalysator nach einem der Ansprüche 1 bis 5, dessen Seitendruckfestigkeit 5 bis 13 N beträgt.

7. Ringförmiger Vollkatalysator nach einem der Ansprüche 1 bis 6, dessen Verhältnis aus scheinbarer Massendichte zu wahrer Massendichte $> 0,55$ beträgt.

8. Verfahren zur Herstellung von Acrolein und/oder Methacrolein durch heterogen katalysierte partielle Gasphasenoxidation von Propen, iso-Buten und/oder tert. Butanol, **dadurch gekennzeichnet, dass** der Katalysator für die Gasphasenoxidation ein ringförmiger Vollkatalysator gemäß einem der Ansprüche 1 bis 7 ist.

9. Verwendung des ringförmigen Vollkatalysators gemäß einem der Ansprüche 1 bis 7 als Katalysator für die heterogen katalysierte partielle Gasphasenoxidation von Propen, iso-Buten und/oder tert. Butanol zur Herstellung von Acrolein und/oder Methacrolein.

**Claims**

1. An annular unsupported catalyst having a curved and/or uncurved top surface of the ring, whose active composition has a stoichiometry of the general formula I

$$Mo_{12}Bi_aFe_bX^1_cX^2_dX^3_eX^4_fO_n \qquad (I)$$

where

X$^1$ = nickel and/or cobalt,
X$^2$ = thallium, an alkali metal and/or an alkaline earth metal,
X$^3$ = zinc, arsenic, boron, antimony, tin, cerium, lead and/or tungsten,
X$^4$ = silicon, aluminum, titanium and/or zirconium,
a = from 0.2 to 5,
b = from 0.01 to 5,
c = from 0 to 10,
d = from 0 to 2,
e = from 0 to 8,
f = from 0 to 10 and
n = a number which is determined by the valency and frequency of the elements in I other than oxygen,
and whose annular geometry, without taking into account any existing curvature of the top surface, has a length L of from 2 to 11 mm, an external diameter E of from 2 to 11 mm and a wall thickness W of from 0.75 mm to 1.75 mm, and wherein the specific surface area S is from 5 to 20 m$^2$/g and the total pore volume V is from 0.1 to 1 cm$^3$/g, the different pore diameters contributing to V as follows:

pores having a diameter in the range from < 0.03 $\mu$m: $\leq$ 5% by volume;
pores having a diameter in the range from $\geq$ 0.03 to $\leq$ 0.1 $\mu$m: $\leq$ 25% by volume;
pores having a diameter in the range from > 0.1 to < 1 $\mu$m: $\geq$ 70% by volume and
pores having a diameter in the range from $\geq$ 1 to $\leq$ 10 $\mu$m: $\leq$ 10% by volume, and which is obtainable by generating a finely divided shapeable mixture from sources of the elemental constituents of the active composition having a particle size which, to an extent of at least 80% by weight of the overall composition, is from 200 $\mu$m to 1.5mm, and, optionally after adding shaping and/or reinforcing assistants, forming from this mixture annular shaped unsupported catalyst precursor bodies having a side crushing strength of $\geq$ 12 N and $\leq$ 23 N and whose top surfaces are curved and/or uncurved, and converting these to the annular unsupported catalysts by thermally treating at elevated temperatures which exceed the temperature of 350 °C and do not exceed the temperature of 650 °C,
where

- Specific surface areas S are determined to DIN 66131 by gas adsorption (N$_2$) according to Brunauer-Emmet-Teller,
- total pore volumes V and also diameter distributions on these total pore volumes are determined by the mercury porosimetry method employing the Auto Pore 9220 instrument from Micromeritics GmbH over a bandwidth from 30 A to 0.3mm, and
- side crushing strengths are determined with a material testing machine for quasistatic stress of the Z 2.5 / TS1S type from Zwick GmbH & Co in D-89079 Ulm with an initial force of 0.5 N, a rate of initial force of 10mm/min and a testing rate of 1.6 mm/min.

2. An annular unsupported catalyst as claimed in claim 1 where S = from 5 to 10 m$^2$/g.

3. An annular unsupported catalyst as claimed in claim 1 or 2 where V = from 0.2 to 0.4 cm$^3$/g.

4. An annular unsupported catalyst as claimed in any of claims 1 to 3, wherein the different pore diameters contribute to V as follows:

pores having a diameter in the range < 0.03 $\mu$m: $\geq$ 0% by volume and $\leq$ 5% by volume;
pores having a diameter in the range $\geq$ 0.03 to $\leq$ 0.1 $\mu$m: $\geq$ 3 and $\leq$ 20% by volume;
pores having a diameter in the range > 0.1 to < 1 $\mu$m: $\geq$ 75 and $\leq$ 95% by volume and
pores having a diameter in the range $\geq$ 1 to $\leq$ 10 $\mu$m: $\geq$ 0 and $\leq$ 5% by volume.

5. An annular unsupported catalyst as claimed in any of claims 1 to 4, wherein the pore diameter d$^{max}$ making the greatest contribution to the total pore volume V is from 0.3 to 0.8 $\mu$m.

6. An annular unsupported catalyst as claimed in any of claims 1 to 5 whose side crushing strength is from 5 to 13 N.

7. An annular unsupported catalyst as claimed in any of claims 1 to 6 whose ratio of apparent mass density to true mass density is > 0.55.

8. A process for preparing acrolein and/or methacrolein by heterogeneously catalyzed partial gas phase oxidation of propene, isobutene and/or tert-butanol, wherein the catalyst for the gas phase oxidation is an annular unsupported catalyst as claimed in any of claims 1 to 7.

9. The use of the annular unsupported catalyst as claimed in any of claims 1 to 7 as catalyst for the heterogeneously catalyzed partial gas phase oxidation of propene, isobutene and/or tert-butanol for preparation of acrolein and/or methacrolein.

**Revendications**

1. Catalyseur massif annulaire à surface frontale incurvée et/ou non incurvée de l'anneau, dont la masse active présente une stoechiométrie de formule générale I

$$Mo_{12}Bi_aFe_bX^1_cX^2_dX^3_eX^4_fO_n \qquad (I)$$

avec

$X^1$ = nickel et/ou cobalt,
$X^2$ = thallium, un métal alcalin et/ou un métal alcalino-terreux,
$X^3$ = zinc, arsenic, bore, antimoine, étain, cérium, plomb et/ou tungstène,
$X^4$ = silicium, aluminium, titane et/ou zirconium,
a = 0,2 à 5,
b = 0,01 à 5,
c = 0 à 10,
d = 0 à 2,
e = 0 à 8,
f = 0 à 10 et
n = un nombre, qui est déterminé par la valence et la fréquence des éléments différents de l'oxygène dans I, et dont la géométrie annulaire présente, sans prendre en compte une courbure éventuelle de la surface frontale, une longueur L de 2 à 11 mm, un diamètre extérieur A de 2 à 11 mm et une épaisseur de paroi W de 0,75 mm à 1,75 mm,
et qui est **caractérisé en ce que** la surface spécifique O est de 5 à 20 $m^2$/g et le volume poreux total V est de 0,1 à 1 $cm^3$/g, les différents diamètres de pores contribuant de la manière suivante à V :

pores d'un diamètre dans la plage < 0,03 $\mu$m : $\leq$ 5 % en volume ;
pores d'un diamètre dans la plage allant de $\geq$ 0,03 à $\leq$ 0,1 $\mu$m : $\leq$ 25 % en volume ;
pores d'un diamètre dans la plage allant de > 0,1 à < 1 $\mu$m : $\geq$ 70 % en volume, et
pores d'un diamètre dans la plage allant de $\geq$ 1 à $\leq$ 10 $\mu$m : $\leq$ 10 % en volume,
et qui peut être obtenu par formation d'un mélange façonnable finement divisé à partir de sources des constituants élémentaires de la masse active, dont la granulométrie est de 200 $\mu$m à 1,5 mm pour au moins 80 % en poids de la masse totale, et façonnage, à partir de ce mélange, éventuellement après ajout d'adjuvants de façonnage et/ou de renforcement, de corps moulés précurseurs de catalyseurs massifs annulaires dont la résistance à la pression latérale est $\geq$ 12 N et $\leq$ 23 N, dont les surfaces frontales sont incurvées et/ou non incurvées, et transformation de ceux-ci par traitement thermique à des températures élevées, qui dépassent la température de 350 °C et ne dépassent pas la température de 650 °C, en les catalyseurs massifs annulaires,

- la surface spécifique O étant déterminée selon DIN 66131 par adsorption de gaz ($N_2$) selon Brunauer-Emmet-Teller,
- les volumes poreux totaux V et les distributions de diamètres sur ces volumes poreux totaux étant déterminés avec la méthode de porosimétrie au mercure en utilisant l'appareil Auto Pore 9220 de la société Micromeritics GmbH sur une largeur de bande de 30 Å à 0,3 mm, et
- les résistances à la pression latérales étant déterminées avec une machine d'essais sur les matériaux pour sollicitation quasi-statique de type Z 2.5/TS1S de la société Zwick GmbH & Co. à D-89079 Ulm avec une pré-force de 0,5 N, une vitesse de pré-force de 10 mm/min et une vitesse d'essai de 1,6 mm/min.

2. Catalyseur massif annulaire selon la revendication 1, avec O = 5 à 10 m$^2$/g.

3. Catalyseur massif annulaire selon la revendication 1 ou 2, avec V = 0,2 à 0,4 cm$^3$/g.

4. Catalyseur massif annulaire selon l'une quelconque des revendications 1 à 3, dans lequel les différents diamètres de pores contribuent de la manière suivante à V :

pores d'un diamètre dans la plage < 0,03 $\mu$m : $\geq$ 0 % en volume et $\leq$ 5 % en volume ;
pores d'un diamètre dans la plage allant de $\geq$ 0,03 à $\leq$ 0,1 $\mu$m : $\geq$ 3 et $\leq$ 20 % en volume ;
pores d'un diamètre dans la plage allant de > 0,1 à < 1 $\mu$m : $\geq$ 75 et $\leq$ 95 % en volume, et
pores d'un diamètre dans la plage allant de $\geq$ 1 à $\leq$ 10 $\mu$m : $\geq$ 0 et $\leq$ 5 % en volume.

5. Catalyseur massif annulaire selon l'une quelconque des revendications 1 à 4, dans lequel le diamètre de pore d$^{max}$ qui apporte la plus grande contribution au volume poreux total V est de 0,3 à 0,8 $\mu$m.

6. Catalyseur massif annulaire selon l'une quelconque des revendications 1 à 5, dont la résistance à la pression latérale est de 5 à 13 N.

7. Catalyseur massif annulaire selon l'une quelconque des revendications 1 à 6, dont le rapport entre la densité massique apparente et la densité massique réelle est > 0,55.

8. Procédé de fabrication d'acroléine et/ou de méthacroléine par oxydation partielle en phase gazeuse sous catalyse hétérogène de propène, d'iso-butène et/ou de tert.-butanol, **caractérisé en ce que** le catalyseur pour l'oxydation en phase gazeuse est un catalyseur massif annulaire selon l'une quelconque des revendications 1 à 7.

9. Utilisation du catalyseur massif annulaire selon l'une quelconque des revendications 1 à 7 en tant que catalyseur pour l'oxydation partielle en phase gazeuse sous catalyse hétérogène de propène, d'iso-butène et/ou de tert.-butanol pour la fabrication d'acroléine et/ou de méthacroléine.

# FIG.1

# FIG.2

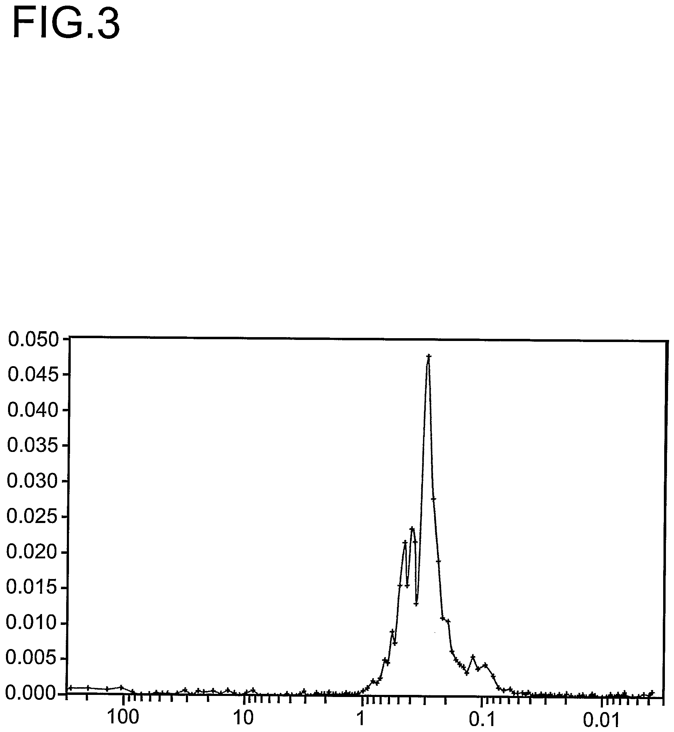

# FIG.3

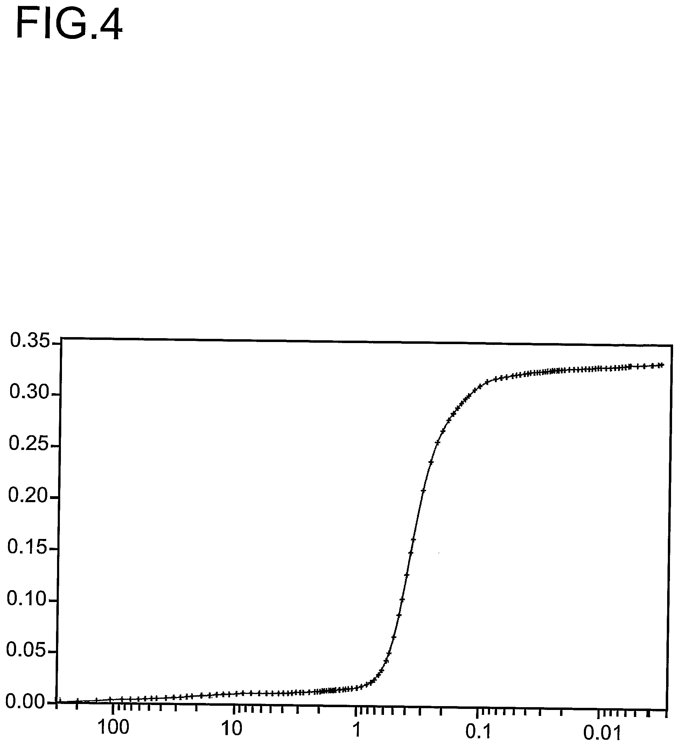

# FIG.4

# FIG.5

# FIG.6

# FIG.7

# FIG.8

# FIG.9

# FIG.10

# FIG.11

# FIG.12

# FIG.13

# FIG.14

# FIG.15

# FIG.16

# FIG.17

# FIG.18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 575897 A
- DE 3300044 A
- DE 19855913 A
- DE 10046957 A
- EP 1340538 A
- DE 19948523 A
- DE 4407020 A
- DE 10101695 A
- US 5082819 A
- DE 10121592 A
- EP 184790 A
- WO 0224620 A
- WO 03039744 A
- EP 279374 A
- WO 0053557 A
- WO 0053558 A
- DE 19910506 A
- EP 1106598 A
- WO 0136364 A
- DE 19927624 A
- DE 19948248 A
- DE 19948241 A
- EP 700714 A
- DE 10313213 A
- DE 10313209 A
- DE 10232748 A
- DE 10313208 A
- DE 3338380 A
- EP 990636 A
- DE 10246119 A
- DE 10245585 A
- DE 4431957 A
- EP 700893 A
- EP 468290 B
- DE 10063162 A
- DE 10337788 A

**In der Beschreibung aufgeführte Nicht-Patentliteratur**

- Ullmann's Encyclopedia of Industrial Chemistry. Electronic Release, 1998